# EUROPEAN PATENT APPLICATION

(11) **EP 4 502 145 A1**
(43) Date of publication of application: **05.02.2025**
(21) Application number: 24192273.1
(22) Date of filing: 01.08.2024
(51) Int. Cl.: C12N 5/077, C12Q 1/6881

(54) **METHOD FOR EVALUATING CELL DIFFERENTIATION STATE, METHOD FOR DETERMINING CELL DIFFERENTIATION STATE, AND METHOD FOR PRODUCING CARDIOMYOCYTES BY DETERMINING MIRNAS IN CULTURE MEDIUM**

(30) Priority: 04.08.2023 JP 2023128004
(71) Applicant: Keio University, Tokyo, 108-8345 (JP); Heartseed Inc., Tokyo 150-0023 (JP); SYSMEX CORPORATION, Kobe-shi Hyogo 651-0073 (JP)
(72) Inventor: Tohyama, Shugo, Tokyo, 160-8582 (JP); Sekine, Otoya, Tokyo, 160-8582 (JP); Kanaami, Sayaka, Tokyo, 150-0023 (JP); Masumoto, Kanako, Hyogo, 651-0073 (JP); Aihara, Yuki, Hyogo, 651-0073 (JP)
(74) Representative: Hoffmann Eitle

(57) **Abstract**

Disclosed is a method for evaluating cell differentiation state, comprising:
inducing differentiation of pluripotent stem cells into mesodermal cells in a liquid medium by a first differentiation treatment for inducing differentiation of pluripotent stem cells into mesodermal cells and a second differentiation treatment for inducing differentiation of the mesodermal cells into cardiomyocytes;
collecting a supernatant of the liquid medium comprising cells induced differentiation by the second differentiation treatment; and
measuring miRNA-3p from miR-1/133a cluster in the supernatant, wherein
the miRNA-3p is at least one selected from a group consisting of miR-1-3p and miR-133a-3p, and
the obtained measurement value of miRNA-3p serves as an index of cell differentiation state of the differentiation-induced cells.

## Description

### FIELD OF THE INVENTION

The present invention relates to a method for evaluating cell differentiation state. The present invention relates to a method for determining cell differentiation state. The present invention relates to a method for producing cardiomyocytes.

### BACKGROUND

In recent years, regenerative medicine using cardiomyocytes obtained by inducing differentiation of pluripotent stem cells has attracted attention as an alternative treatment for heart transplantation. However, it is difficult to always acquire a large amount of cardiomyocytes with high efficiency by differentiation induction. For example, the differentiation efficiency into cardiomyocytes varies due to an influence such as a difference in quality of pluripotent stem cells to be used. In order to apply cardiomyocytes obtained by differentiation induction to a patient, it is necessary to evaluate cell differentiation state. For the evaluation of the differentiation state from pluripotent stem cells into cardiomyocytes, cardiac troponin T (cTnT), which is a marker for cardiomyocytes, has been conventionally used. In this evaluation method, first, a part of cells after differentiation induction is collected and immunostained with a fluorescently labeled anti-cTnT antibody. Then, cells expressing cTnT are detected by flow cytometry, and the proportion of cells expressing cTnT among cells after differentiation induction (cTnT positive rate) is calculated. Conventionally, the cell differentiation state into cardiomyocytes has been evaluated based on the cTnT positive rate. However, since the fluorescent-labeled cells cannot be used for transplantation into a living body, this evaluation method can be referred to as destructive test. As pluripotent stem cells and cells prepared therefrom are valuable, it is not preferred to consume some for quality assessment. Meanwhile, as an example of non-destructive test, Zhang Y. et al., A non-invasive method to determine the pluripotent status of stem cells by culture medium microRNA expression detection (2016) Scientific Reports, vol. 6: 22380 discloses that the cell differentiation state has been evaluated by measuring the quantity of miRNA in a culture supernatant in the process of inducing differentiation of human embryonic stem cells into cardiomyocytes.

### SUMMARY OF THE INVENTION

In Zhang Y. et al., A non-invasive method to determine the pluripotent status of stem cells by culture medium microRNA expression detection (2016) Scientific Reports, vol. 6: 22380, in order to evaluate the cell differentiation state of human embryonic stem cells into cardiomyocytes, miRNAs known to be expressed only in mice and miRNAs known to have a small expression level have been also measured. Therefore, there is a concern about the accuracy of the evaluation of the cell differentiation state. An object of the present invention is to provide a means capable of evaluating cell differentiation state with higher accuracy.

The present inventors have found that the cell differentiation state can be evaluated by measuring miRNA-3p from miR-1/133a cluster in a supernatant of a liquid medium containing differentiation-induced cells, thereby completing the present invention. Therefore, the following inventions [1] to [24] are provided.
[1] A method for evaluating cell differentiation state, including inducing pluripotent stem cells to differentiate into cardiomyocytes in a liquid medium by a first differentiation treatment for inducing pluripotent stem cells to differentiate into mesodermal cells treatment and a second differentiation treatment for inducing differentiating the mesodermal cells to differentiate into cardiomyocytes treatment; collecting a supernatant of the liquid medium containing cells induced to differentiate by the second differentiation treatment; and measuring miRNA-3p in miR-1/133a cluster in the supernatant, in which the miRNA-3p is at least one selected from a group consisting of miR-1-3p and miR-133a-3p, and the measured value of miRNA-3p is an index of differentiation into cardiomyocytes.
[2] The method according to [1], in which the miRNA-3p is the miR-1-3p, and when the measured value of miR-1-3p is less than a first threshold value, it is suggested that the differentiation-induced cells are not applicable for transplantation.
[3] The method according to [1], in which the miRNA-3p is the miR-133a-3p, and when the measured value of miR-133a-3p is less than a second threshold value, it is suggested that the differentiation-induced cells are not applicable for transplantation.
[4] The method according to [1], in which the miRNA-3p is the miR-1-3p and the miR-133a-3p, and when the measured value of miR-1-3p is less than a first threshold value and the measured value of miR-133a-3p is less than a second threshold value, it is suggested that the differentiation-induced cells are not applicable for transplantation.
[5] The method according to [1], in which the miRNA-3p is the miR-1-3p and the miR-133a-3p, and when the measured value of miR-1-3p is less than a first threshold value or the measured value of miR-133a-3p is less than a second threshold value, it is suggested that the differentiation-induced cells are not applicable for transplantation.
[6] The method according to any one of [1] to [5], in which the first differentiation treatment includes using a liquid medium containing at least one of a substance that activates a BMP signaling system or a substance that activates a Wnt signaling system.
[7] The method according to any one of [1] to [6], in which the second differentiation treatment includes using a liquid medium containing a substance that inhibits a Wnt signaling system.
[8] The method according to any one of [1] to [7], further including collecting a supernatant of the liquid medium containing cells induced to differentiate by the first differentiation treatment and before being subjected to the second differentiation treatment, and measuring miR-489-3p in the supernatant, in which the measured value of miR-489-3p is an index of differentiation into mesodermal cells.
[9] The method according to [8], in which when the measured value of miR-489-3p is equal to or more than a third threshold value, it is suggested that the pluripotent stem cells have differentiated into mesodermal cells.
[10] A method for determining cell differentiation state, including a differentiation inducing including a first differentiation treatment for inducing pluripotent stem cells to differentiate into mesodermal cells in a liquid medium and a second differentiation treatment for inducing the mesodermal cells to differentiate into cardiomyocytes; collecting a supernatant of the liquid medium containing cells induced to differentiate by the second differentiation treatment; measuring miRNA-3p in miR-1/133a cluster in the supernatant; and determining that the differentiation-induced cells are not applicable for transplantation when the measured value of miRNA-3p is less than a threshold value, in which the miRNA-3p is at least one selected from miR-1-3p and miR-133a-3p.
[11] The method according to [10], in which the miRNA-3p is the miR-1-3p, and the threshold value is a first threshold value, and in the determining, when the measured value of miR-1-3p is less than the first threshold value, it is determined that the differentiation-induced cells are not applicable for transplantation.
[12] The method according to [10], in which the miRNA-3p is the miR-133a-3p, and the threshold value is a second threshold value, and in the determining, when the measured value of miR-133a-3p is less than the second threshold value, it is determined that the differentiation-induced cells are not applicable for transplantation.
[13] The method according to [10], in which the miRNA-3p is the miR-1-3p and the miR-133a-3p, and the threshold values are a first threshold value and a second threshold value, and in the determining, when the measured value of miR-1-3p is less than the first threshold value and the measured value of miR-133a-3p is less than the second threshold value, it is determined that the differentiation-induced cells are not applicable for transplantation.
[14] The method according to [10], in which the miRNA-3p is the miR-1-3p and the miR-133a-3p, and the threshold values are a first threshold value and a second threshold value, and in the determining, when the measured value of miR-1-3p is less than the first threshold value or the measured value of miR-133a-3p is less than the second threshold value, it is determined that the differentiation-induced cells are not applicable for transplantation.
[15] The method according to any one of [10] to [14], in which the first differentiation treatment includes differentiation treatment using a liquid medium containing at least one of a substance that activates a BMP signaling system or a substance that activates a Wnt signaling system.
[16] The method according to any one of [10] to [15], in which the second differentiation treatment includes using a liquid medium containing a substance that inhibits a Wnt signaling system.
[17] The method according to any one of [10] to [16], further including collecting a supernatant of the liquid medium containing cells induced to differentiate by the first differentiation treatment and before being subjected to the second differentiation treatment, and measuring miR-489-3p in the supernatant; and
   determining that the pluripotent stem cells have differentiated into mesodermal cells when the measured value of miR-489-3p is equal to or more than a third threshold value.
[18] The method according to any one of [10] to [17], further including collecting a supernatant of the liquid medium containing cells induced to differentiate by the first differentiation treatment and before being subjected to the second differentiation treatment, and measuring miR-489-3p in the supernatant; and determining that differentiation of the pluripotent stem cells into mesodermal cells is insufficient when the measured value of miR-489-3p is less than a third threshold value.
[19] A method for producing cardiomyocytes including (1) inducing mesodermal cells to differentiate into cardiomyocytes in a liquid medium by differentiation treatment for inducing mesodermal cells to differentiate into cardiomyocytes; (2) collecting a supernatant of the liquid medium containing cells induced in the step (1); (3) measuring miRNA-3p in miR-1/133a cluster in the supernatant collected in the step (2); and (4) culturing the cells induced to differentiate in the step (1) to acquire cardiomyocytes when the measured value of miRNA-3p is equal to or more than a threshold value, in which the miRNA-3p is at least one selected from miR-1-3p and miR-133a-3p.
[20] The method according to [19], in which in the step (4), when the measured value of miRNA-3p is less than a threshold value, culture of the cells induced to differentiate in the step (1) is stopped.
[21] The production method according to [19] or [20], including (i) inducing pluripotent stem cells to differentiate into mesodermal cells in a liquid medium by differentiation treatment for inducing pluripotent stem cells to differentiate into mesodermal cells; (ii) collecting a supernatant of the liquid medium containing cells induced in the step (i); and (iii) measuring miR-489-3p in the supernatant collected in the step (ii), in which when the measured value of miR-489-3p is equal to or more than a third threshold value, the cells of the step (ii) are used as the mesodermal cells of the step (1).
[22] The method according to [21], in which when the measured value of miR-489-3p is less than a threshold value, culture of the cells of the step (ii) is stopped.
[23] A reagent kit for use in the method according to any one of [1] to [7], [10] to [16], [19], and [20], including an oligonucleotide primer and/or an oligonucleotide probe for detecting miRNA-3p in miR-1/133a cluster.
[24] A reagent kit for use in the method according to any one of [8], [9], [17], [18], [21], and [22], including an oligonucleotide primer and/or an oligonucleotide probe for detecting miRNA-3p in miR-1/133a cluster; and an oligonucleotide primer and/or an oligonucleotide probe for detecting miR-489-3p.

According to the present invention, it is possible to accurately evaluate cell differentiation state.

### BRIEF DESCRIPTION OF THE DRAWINGS

Fig. 1 is a diagram showing an example of a schedule of differentiation induction and acquisition timing of supernatant.
Fig. 2A is a diagram showing an example of an appearance of a reagent kit.
Fig. 2B is a diagram showing an example of an appearance of a reagent kit.
Fig. 3 is a diagram showing an example of an appearance of a reagent kit.
Fig. 4 is a diagram showing a schedule of differentiation induction and acquisition timings of supernatant in Example 1.
Fig. 5A is a graph showing copy number of miR-489-3p in a supernatant obtained in a process of inducing differentiation of iPS cells of 201B7 clone. Group H is cells subjected to the first and second differentiation treatments, and group L is cells subjected to only the second differentiation treatment.
Fig. 5B is a graph showing copy number of miR-489-3p in a supernatant obtained in a process of inducing differentiation of iPS cells of 253G4 clone.
Fig. 6 is an example of images of cells subjected to immunofluorescence staining using an anti-brachyury-T antibody and a fluorescently labeled secondary antibody for Groups Hand L.
Fig. 7 is a graph showing the ratio of brachyury T-positive cells in Groups H and L.
Fig. 8 is a graph with fluorescence signal intensity (in the figure, denoted as "cTnT") and count (number of cells) as biaxes for Groups Hand L.
Fig. 9A is a box plot showing relative expression level of miR-1-3p in the supernatant. Fig. 9B is a box plot showing relative expression level of miR-1-5p in the supernatant. The passed product is cells of lots having a cTnT positive rate of 70% or more on day 10 (d10 in Fig. 4). The "failed product" is cells of lots having a cTnT positive rate of less than 70% on day 10.
Fig. 10 is a box plot obtained by converting the relative expression level in Fig. 9A into copy number.
Fig. 11A is a box plot showing relative expression level of miR-133a-3p in the supernatant. Fig. 11B is a box plot showing relative expression level of miR-133a-5p in the supernatant.
Fig. 12 is a box plot obtained by converting the relative expression level in Fig. 11A into copy number.
Fig. 13A is a box plot showing relative expression level of miR-23b-3p in the supernatant. Fig. 13B is a box plot showing relative expression level of miR-23b-5p in the supernatant.
Fig. 14 is a box plot re-created from the box plot in Fig. 13A by changing the maximum value on the vertical axis to 80.
Fig. 15A is a box plot showing relative expression level of miR-26b-3p in the supernatant. Fig. 15B is a box plot showing relative expression level of miR-26b-5p in the supernatant.
Fig. 16 is a box plot re-created from the box plot in Fig. 15 by changing the maximum value on the vertical axis to 80.
Fig. 17A is a box plot showing relative expression level of miR-125b-3p in the supernatant. Fig. 17B is a box plot showing relative expression level of miR-125b-5p in the supernatant.
Fig. 18A is a box plot showing relative expression level of miR-145-3p in the supernatant. Fig. 18B is a box plot showing relative expression level of miR-145-5p in the supernatant.
Fig. 19A is a box plot showing relative expression level of miR-499a-3p in the supernatant. Fig. 19B is a box plot showing relative expression level of miR-499a-5p in the supernatant.
Fig. 20A is a box plot showing relative expression level of miR-503-3p in the supernatant. Fig. 20B is a box plot showing relative expression level of miR-503-5p in the supernatant.

### DETAILED DESCRIPTION OF THE PREFERRED EMBODIMENTS

In the method for evaluating cell differentiation state of the present embodiment (hereinafter, also referred to as "the evaluation method of the present embodiment"), first, pluripotent stem cells are induced to differentiate into cardiomyocytes in a liquid medium by a first differentiation treatment for inducing pluripotent stem cells to differentiate into mesodermal cells in a liquid medium and a second differentiation treatment for inducing the mesodermal cells to differentiate into cardiomyocytes.

Pluripotent stem cells (PSC) are stem cells having an ability to differentiate into cells derived from ectoderm, endoderm and mesodermal (pluripotency) and having proliferation capability. Examples of PSCs include induced pluripotent stem cells (iPS cells), embryonic stem cells (ES cells), nuclear transplanted ES cells (ntES cells), embryonic germ stem cells (EG cells), and the like. The iPS cell is prepared by introducing a predetermined reprogramming factor (DNA or protein) such as Oct3/4, Klf4, c-Myc, or Sox2 into a somatic cell, and is a stem cell having pluripotency and proliferation capability. The ES cell is a stem cell derived from inner cell mass of a mammalian blastocyst, and is a stem cell having pluripotency and proliferation capability. The ntES cell is an ES cell established from blastocyst inner cell mass derived from a clone embryo obtained by replacing the nucleus of an unfertilized egg with the nucleus of a somatic cell. The ntES cell has almost the same properties as the ES cell. The EG cell is a cell established from an embryonic primordial germ cell and has pluripotency similar to that of ES cell. The EG cell may be established by culturing primordial germ cells in the presence of substances such as LIF, basic FGF (bFGF), stem cell factor, and the like. Among them, the iPS cell is preferable.

The origin of PSC is not particularly limited. For example, PSC is preferably derived from a mammal such as human, monkey, dog, cat, horse, mouse, rat, hamster, guinea pig, rabbit, sheep, pig, or cow. From the viewpoint of regenerative medicine research and clinical application, human PSC (hPSC) is preferable as the PSC.

The liquid medium itself is known, and can be appropriately selected according to the type of PSC, for example. Examples of the liquid medium include RPMI1640 medium, StemFit (trademark) series (AJINOMOTO CO., INC.), MEM α medium, IMDM medium, Medium 199 medium, EMEM medium, DMEM medium, Ham's F 12 medium, Fischer's medium, MEF-CM, StemPro (trademark) 34 (Invitrogen), Essential 8 (trademark) (Thermo Fisher Scientific), ReproNaive (trademark) (ReProcell Inc.), hPSC Growth Medium DXF (trademark) (Takara Bio Inc.), mixed media thereof, and the like.

The liquid medium may contain components necessary for growth and/or maintenance of PSCs. Examples of such components include serum, albumin, transferrin, B-27 (trademark) supplement (Invitrogen), alternative serum (Knockout (trademark) Serum Replacement) (KSR), N2 supplement (Invitrogen), fatty acid, insulin, collagen precursor, 2-mercaptoethanol, thiol glycerol, lipid, amino acid, L-glutamine, Glutamax (trademark) (Invitrogen), non-essential amino acid, vitamin, growth factor, antibiotic, antioxidant, pyruvic acid, buffer, inorganic salt, and the like.

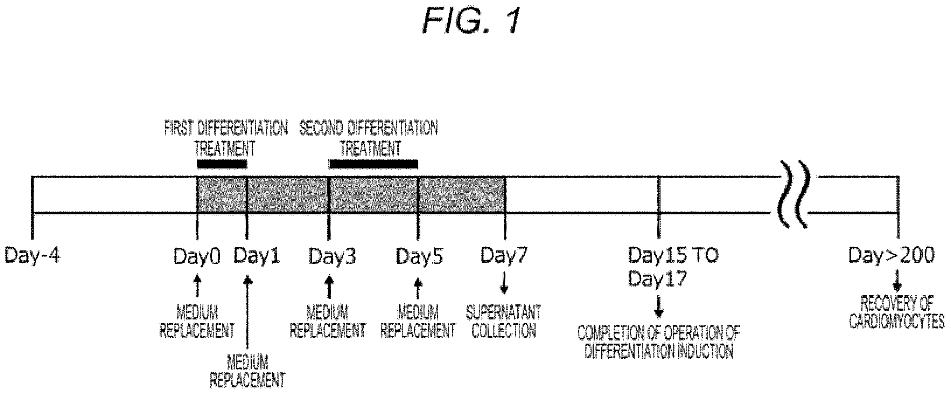

With reference to Fig. 1, the schedule of cell culture and differentiation induction and the timing of acquiring supernatant in the evaluation method of the present embodiment will be described. However, the present invention is not limited to the example of Fig. 1. In the example of Fig. 1, the day on which the first differentiation treatment was performed is defined as "day 0". In Fig. 1, day 0 is denoted as "Day0". Fig. 1 shows on which day the operations performed before and after the first differentiation treatment are performed with reference to day 0. In the figure, a period indicated in gray (Day0 to Day7) is an example of a period during which the evaluation method of the present embodiment is performed.

The PSC used in the evaluation method of the present embodiment is preferably maintained and cultured in a liquid medium in advance before performing the first differentiation treatment. The maintenance culture refers to culturing PSCs until they proliferate to the number of cells applicable for differentiation induction while maintaining pluripotency. The period of maintenance culture is not particularly limited, and is, for example, 1 day or more and 7 days or less. In the example of Fig. 1, the maintenance culture period is 4 days from 4 days before day 0 (Day-4) to day 0.

The liquid medium used for maintenance culture preferably contains components necessary for survival and/or maintenance of undifferentiated PSCs. Examples of the component include a ROCK inhibitor (for example, Y-27632), a leukemia inhibitory factor (LIF), a fibroblast growth factor (FGF), a stem cell factor, a MEK inhibitor (for example, PD184352), and the like. When the PSC is an iPS cell, it is preferable to use a liquid medium containing a ROCK inhibitor on the first day of maintenance culture. By adding the ROCK inhibitor, an effect of reducing cell death when iPS cells are suspended and seeded in a liquid medium is obtained. At a time point at least 1 to 4 days after the start of maintenance culture of PSCs in a liquid medium containing a ROCK inhibitor, the liquid medium is preferably replaced with a liquid medium containing no ROCK inhibitor.

The first differentiation treatment is performed by adding to PSCs in the liquid medium, a substance that activates differentiation induction from PSCs into mesodermal cells. Such a substance is known per se. For example, in the first differentiation treatment, a liquid medium containing at least one of a substance that activates a bone morphogenetic protein (BMP) signaling system or a substance that activates a Wnt signaling system is used. Specifically, the liquid medium containing cells (PSCs in this case), is replaced with a liquid medium containing at least one of these substances. Alternatively, at least one of these substances may be added to a liquid medium containing cells. When these substances are bound to receptors of PSCs and/or incorporated into PSCs in the liquid medium, the PSCs are induced to differentiate into mesodermal cells.

In the present specification, the "liquid medium containing cells" refers to a liquid medium in contact with cells or in which cells are immersed in a culture vessel. In the liquid medium containing cells, the cells may be suspended in the liquid medium, or may be settled or attached to the bottom of the culture vessel in the liquid medium.

The substance that activates a BMP signaling system is not particularly limited, and examples thereof include BMP4, BMP2, activin, and the like. The substance that activates a Wnt signaling system is not particularly limited, and examples thereof include a Wnt signal agonist, activin A, BMP-4, bFGF, and the like. Examples of the Wnt signal agonist include a glycogen synthase kinase (GSK)-3 inhibitor and the like. The GSK-3 inhibitor itself is known, and examples thereof include CHIR99021, TWS119, SB2167363, SB415286, CHIR-98014, and the like.

The timing to start the first differentiation treatment is not particularly limited. When PSCs proliferate to the number of cells applicable for differentiation induction while maintaining pluripotency, the first differentiation treatment may be performed. In the example of Fig. 1, the first differentiation treatment is performed 3 or 4 days after the start of the maintenance culture. The period of the first differentiation treatment is not particularly limited, but is, for example, 12 hours or more and 6 days or less, and preferably 1 day or more and 6 days or less. In the example of Fig. 1, the first differentiation treatment is performed until day 1 (Day1). The first differentiation treatment can be completed by replacing the liquid medium containing cells with a liquid medium containing no substance that activates a BMP signaling system and substance that activates a Wnt signaling system. Although the first differentiation treatment itself is completed by medium replacement, PSCs differentiate into mesodermal cells even after medium replacement. When the period of the first differentiation treatment is determined by the number of days, the time at which the first differentiation treatment is completed is preferably the time at which the first differentiation treatment is started ±1 hour.

The second differentiation treatment is performed by adding to the cells in the liquid medium after the first differentiation treatment, a substance that activates differentiation induction from mesodermal cells into cardiomyocytes. Such a substance is known per se. For example, in the second differentiation treatment, a liquid medium containing a substance that inhibits a Wnt signaling system is used. Specifically, the liquid medium containing cells is replaced with a liquid medium containing the substance. Alternatively, the substance may be added to the liquid medium containing cells. When the substance that inhibits a Wnt signaling system is taken into cells in the liquid medium, the cells are induced to differentiate into cardiomyocytes.

The substance that inhibits a Wnt signaling system is not particularly limited, and examples thereof include a substance that activates decomposition of β-catenin. Such substances are known per se and include, for example, IWR-1, IWP-2, IWP-3, IWP-4, PNU-74654, XAV939, KY02111, and the like.

The timing to start a second differentiation treatment can be appropriately determined. If a sufficient period of time has elapsed for PSCs to differentiate into mesodermal cells, the second differentiation treatment may be started. Preferably, the second differentiation treatment is started at a time point at which 2 days or more and 7 days or less have elapsed from the day on which the first differentiation treatment was started. In the example of Fig. 1, the second differentiation treatment is started on day 3 (Day3), that is, 3 days after the day on which the first differentiation treatment was started. The period of the second differentiation treatment is not particularly limited, and is, for example, 12 hours or more and 5 days or less. In the example of Fig. 1, the second differentiation treatment is performed from day 3 (Day3) to day 5 (Day5). The second differentiation treatment can be completed by replacing the liquid medium with a liquid medium containing no substance that inhibits a Wnt signaling system. Although the second differentiation treatment itself is completed by medium replacement, mesodermal cells are induced to differentiate into cardiomyocytes even after medium replacement. When the period of the second differentiation treatment is determined by the number of days, the time at which the second differentiation treatment is completed is preferably the time at which the second differentiation treatment is started ± 1 hour.

The method for culturing cells in the period of maintenance culture and differentiation induction can be appropriately selected from known culturing methods depending on the type of PSC. For culturing cells, suspension culture, adhesion culture, or a combination thereof is usually used. The suspension culture refers to culture performed under a non-adhesive condition to a culture vessel. In suspension culture, cells do not necessarily need to be dispersed in a liquid medium, and cells may be settled at the bottom of the culture vessel. The culture vessel used for suspension culture is not particularly limited, and examples thereof include a flask, a dish, a plate, a chamber, a tube, and the like. The culture vessel used for suspension culture is preferably cell-non-adherent. The culture vessel is preferably made of a hydrophobic material or coated on its surface with a coating agent for preventing adhesion of cells (for example, a polyhydroxyethyl methacrylate copolymer or the like).

The adhesion culture refers to culture performed by adhering cells to a support. As the support, a culture vessel is used. The culture vessel used for adhesion culture is not particularly limited. For adhesion culture, the culture vessel used for suspension culture can be used, but it is preferable that the surface or inside of the culture vessel is coated with a coating agent for adhering cells (also called cell culture substrate, adhesion substrate, or the like). Examples of the coating agent include gelatin, laminin, collagen, poly-D-lysine, polyornithine, fibronectin, vitronectin, and the like. As the support, a known scaffold can also be used. By using this, PSCs grow on the scaffold, and three-dimensional culture can be performed. As such a scaffold, a commercially available product can be used, and examples thereof include Matrigel (trademark) (Corning), QGel (trademark) MT 3D Matrix (Qgel SA), 3-D Life Biomimetic (Cellendes), Puramatrix (3D MATRIX), alvetex (reinnavate), and the like.

As necessary, the PSCs may be cultured with feeder cells. The type of feeder cells is not particularly limited, and can be appropriately selected from known feeder cells. Examples thereof include mouse embryonic fibroblasts (MEF), mouse embryonic fibroblast lines (STO), SL10 cells, SNL cells, and the like.

The culture conditions of PSCs can be appropriately adjusted according to the type of PSC. For example, iPS cells can be cultured in a multi-gas incubator at a CO₂ concentration and an O₂ concentration of 1% or more and 10% or less, preferably 5%, at a temperature of about 30°C or more and 40°C or less, preferably 37°C. After differentiation into cardiomyocytes, the cells can be cultured in a CO₂ incubator at a CO₂ concentration of 1% or more and 10% or less, preferably 5%, at a temperature of about 30°C or more and 40°C or less, preferably 37°C.

In the evaluation method of the present embodiment, a supernatant of the liquid medium containing cells induced to differentiate by the second differentiation treatment is collected. The supernatant is all or a part of a solution portion of the liquid medium containing cells, and is substantially free of cells. The method for collecting the supernatant from the liquid medium containing cells is not particularly limited. In the case of suspension culture, the supernatant after centrifugation can be obtained by centrifuging the liquid medium containing cells. In the case of adhesion culture, since the cells adhere to the bottom of the culture vessel, the supernatant can be obtained by aspirating the supernatant in the culture vessel with a pipette or the like. The collected supernatant is used as a sample for miRNA measurement described later.

In the evaluation method of the present embodiment, miRNA in the supernatant secreted from the cells is to be measured. When the cells are mixed in the collected supernatant, it is preferable to remove the cells from the supernatant in order to avoid measurement of miRNA in the cells. Removal of cells from the supernatant can be performed by filter filtration and/or centrifugation.

As shown in Fig. 1, it is known that it usually takes 15 to 17 days from the day on which the first differentiation treatment was started until the operation of inducing differentiation into cardiomyocytes is completed. In the present specification, the "operation of inducing differentiation into cardiomyocytes" includes performing the first and second differentiation treatments and culturing the cells for a period of time sufficient for the cells in the liquid medium to terminally differentiate into cardiomyocytes. Conventionally, the cTnT positive rate of cells has been confirmed after completion of the operation of inducing differentiation into cardiomyocytes. However, the evaluation method of the present embodiment can evaluate the cell differentiation state after completion of differentiation induction before completion of the operation of inducing differentiation into cardiomyocytes. For example, in the example of Fig. 1, the cell differentiation state can be evaluated by the measured value of miRNA in the supernatant collected at Day7. In the present specification, the "cells induced to differentiate by the second differentiation treatment" refers to cells after being induced to differentiate by the second differentiation treatment and before the operation of inducing differentiation into cardiomyocytes is completed.

As described above, in the evaluation method of the present embodiment, the cell differentiation state can be evaluated by the measured value of miRNA in the supernatant collected before completion of the operation of inducing differentiation into cardiomyocytes. Therefore, the collection timing of the supernatant may be before the operation of inducing differentiation into cardiomyocytes is completed. On the other hand, it is considered that miRNA-3p in miR-1/133a cluster is not sufficiently secreted into the supernatant even if the supernatant is collected when the time has not elapsed so much from the day when the second differentiation treatment was performed. The second differentiation treatment is completed by medium replacement. Even when the time has not elapsed so much after medium replacement, it is considered that the miRNA-3p is not sufficiently contained in the supernatant. From these, the collection timing of the supernatant may be, for example, a time point at which 3 days or more and 4 days or less have elapsed from the day on which the second differentiation treatment was performed and before the second differentiation treatment is completed. The time point before completing the second differentiation treatment includes, for example, a time point one day before completing the second differentiation treatment or a time point at which the old liquid medium is removed for medium replacement. Alternatively, the collection timing of the supernatant may be a time point at which 2 days or more and 3 days or less have elapsed from the day on which the second differentiation treatment was completed.

The timing of collecting the supernatant may be determined, for example, according to the length of the period of the second differentiation treatment. When the period of the second differentiation treatment is 12 hours or more and less than 24 hours, the collection timing of the supernatant may be, for example, a time point at which 3 days or more and 4 days or less have elapsed from the day on which the second differentiation treatment was completed. When the period of the second differentiation treatment is 1 day or 2 days, the collection timing of the supernatant may be, for example, a time point at which 2 days or more and 3 days or less have elapsed from the day on which the second differentiation treatment was completed. When the period of the second differentiation treatment is 3 days or more, the collection timing of the supernatant may be, for example, a time point at which 3 days or more and 4 days or less have elapsed from the day on which the second differentiation treatment was started and before the second differentiation treatment is completed. In the example of Fig. 1, the period of the second differentiation treatment is 2 days, and the supernatant is collected on day 7 (Day7), that is, 2 days after the completion of the second differentiation treatment.

As a negative control for the measurement of miRNA-3p in miR-1/133a cluster, a supernatant before the second differentiation treatment is performed (hereinafter also referred to as "second control sample") may be collected. The second control sample may be a supernatant collected at any time before the second differentiation treatment is started. The supernatant collected at such a time point contains little miRNA-3p in miR-1/133a cluster. The control sample can also be said to be a sample for obtaining a background value for the measurement of the miRNA-3p. Preferably, the second control sample is a liquid medium that is removed upon medium replacement to start the second differentiation treatment. In the example of Fig. 1, the second control sample is a liquid medium containing cells before medium replacement on day 3 (Day3). The second control sample can be obtained by recovering a part or all of the liquid medium before removing the liquid medium.

In the evaluation method of the present embodiment, miRNA-3p in miR-1/133a cluster in the supernatant is measured. The miRNA-3p is a mature miRNA expressed from the 3'-terminal side of pre-miRNA which is a precursor of miRNA. The mature miRNA expressed from the 5'-terminal side of pre-miRNA is called miRNA-5p. A gene group encoding human miR-1-3p and miR-133a-3p is present on chromosomes 18 and 20, and forms miR-1/133a cluster. The miRNA-3p in miR-1/133a cluster is miR-1-3p and miR-133a-3p. As used herein, the "miRNA-3p in miR-1/133a cluster" refers to at least one selected from miR-1-3p and miR-133a-3p. That is, measuring miRNA-3p in miR-1/133a cluster refers to measuring miR-1-3p and/or miR-133a-3p.

Human miR-1-3p and human miR-133a-3p themselves are known, and the nucleotide sequences of these miRNAs are shown in SEQ ID NOs: 1 and 2, respectively. Genes encoding these miRNAs are each disclosed in GenBank database as shown in Table 1.

**[Table 1]**

| Gene | GenBank Accession No. |
|---|---|
| miR-1 Gene | NR_029780.1 |
| miR-133a Gene | NR_029675.1 |

The miRNA measurement method is not particularly limited, and a known method can be used. Examples thereof include a measurement method using oligonucleotide probe hybridization, a measurement method using nucleic acid amplification, a measurement method using mass spectrometry, a measurement method using sequencing, and the like. Examples of the measurement method using probe hybridization include microarray, Northern hybridization, RNase protection assay, and the like. Examples of the measurement method using nucleic acid amplification include quantitative RT-PCR, quantitative RT-LAMP, and the like. Examples of the quantitative RT-PCR include SYBR (trademark) Green, TaqMan (trademark), and the like. Since the miRNA to be amplified is short, a stem-loop primer, poly (A) addition, or the like is usually used in a method using nucleic acid amplification. A commercially available reagent kit for miRNA quantification such as TaqMan (trademark) MicroRNA Assay (Thermo Fisher Scientific) may be used.

The measured value of miRNA-3p in miR-1/133a cluster is an index of differentiation into cardiomyocytes. The measured value of miRNA-3p in miR-1/133a cluster is a measured value of miR-1-3p and/or a measured value of miR-133a-3p. Hereinafter, the "measured value of miRNA-3p in miR-1/133a cluster" is also referred to as the "measured value of miRNA-3p". In the present specification, the "measured value" is a value reflecting the abundance of miRNA in the supernatant. Examples thereof include an optical measured value, the number of reaction cycles (Ct value) or reaction time when the optical measured value reaches a predetermined reference value, a quantitative value of miRNA calculated using a calibration curve, and the like. The "measured value" may be a difference from or ratio to a background value. Examples of the optical measured value include fluorescence intensity, turbidity, absorbance, and the like. Examples of the quantitative value of miRNA include copy number, mass, concentration, and the like. The predetermined reference value can be appropriately set according to the type of value indicating the abundance or concentration of miRNA. For example, when the quantitative RT-PCR is used, the amount of change in fluorescence intensity when the nucleic acid amplification reaction exhibits logarithmic amplification can be set using a predetermined Ct value as a reference value.

As shown in Example 2 described later, the measured value of miRNA-3p in the supernatant collected before completion of the operation of inducing differentiation into cardiomyocytes was significantly higher in cells having a cTnT positive rate of 90% or more than in cells having a cTnT positive rate of less than 70% after completion of the operation (d10). Therefore, based on the measured value of miRNA-3p, information on the cell differentiation state when the differentiation-inducing operation was completed can be acquired for cells induced to differentiate by the second differentiation treatment. Such information may be, for example, information suggesting whether the cells induced to differentiate by the second differentiation treatment can be applied to transplantation as cardiomyocytes after completion of the differentiation-inducing operation. The phrase "can be applied to transplantation" means a state in which transplantation into a living body becomes possible if at least one of the criteria for determining that transplantation is possible is satisfied and all the other criteria are satisfied. According to the method, even if it is suggested that the cells "can be applied to transplantation", it may be determined that transplantation is impossible according to other criteria. One of the indices of whether cardiomyocytes prepared by differentiation induction of PSCs can be applied to transplantation is the ratio of the degree to which cells induced to differentiate by the second differentiation treatment differentiate into cardiomyocytes after completion of the differentiation-inducing operation. Therefore, the information regarding the cell differentiation state when the differentiation-inducing operation was completed may be, for example, a suggestion that at least 70%, at least 75%, at least 80%, at least 85%, at least 90%, or at least 95% of the cells induced to differentiate by the second differentiation treatment differentiate into cardiomyocytes.

For example, in a case where the measured value of miR-1-3p is acquired as the measured value of miRNA-3p, when the measured value of miR-1-3p is less than a first threshold value, it can be suggested that the cells induced to differentiate by the second differentiation treatment are not applicable for transplantation. When the measured value of miR-1-3p is equal to or more than the first threshold value, it can be suggested that the cells induced to differentiate by the second differentiation treatment can be applied to transplantation. When the measured value of miR-1-3p is equal to or more than the first threshold value, it can be suggested that at least 70% of the cells induced to differentiate by the second differentiation treatment differentiate into cardiomyocytes. The first threshold value is a threshold value corresponding to the measured value of miR-1-3p. Alternatively, in a case where the measured value of miR-133a-3p is acquired as the measured value of miRNA-3p, when the measured value of miR-133a-3p is less than a second threshold value, it can be suggested that the cells induced to differentiate by the second differentiation treatment are not applicable for transplantation. When the measured value of miR-133a-3p is equal to or more than the second threshold value, it can be suggested that the cells induced to differentiate by the second differentiation treatment can be applied to transplantation. When the measured value of miR-133a-3p is equal to or more than the second threshold value, it can be suggested that at least 70% of the cells induced to differentiate by the second differentiation treatment differentiate into cardiomyocytes. The second threshold value is a threshold value corresponding to the measured value of miR-133a-3p. By the above evaluation method using the measured value of miR-1-3p or the measured value of miR-133a-3p, it is possible to early identify cells unapplicable for transplantation or cells applicable to transplantation, and to early determine whether or not culture can be continued.

Alternatively, in a case where the measured value of miR-1-3p and the measured value of miR-133a-3p are acquired as the measured values of miRNA-3p, when the measured value of miR-1-3p is less than the first threshold value and the measured value of miR-133a-3p is less than the second threshold value, it can be suggested that the cells induced to differentiate by the second differentiation treatment are not applicable for transplantation. When the measured value of miR-1-3p is equal to or more than the first threshold value and the measured value of miR-133a-3p is equal to or more than the second threshold value, it can be suggested that the cells induced to differentiate by the second differentiation treatment can be applied to transplantation. When the measured value of miR-1-3p is equal to or more than the first threshold value and the measured value of miR-133a-3p is equal to or more than the second threshold value, it can be suggested that at least 70% of the cells induced to differentiate by the second differentiation treatment differentiate into cardiomyocytes.

Alternatively, in a case where the measured value of miR-1-3p and the measured value of miR-133a-3p are acquired as the measured values of miRNA-3p, when the measured value of miR-1-3p is less than the first threshold value or the measured value of miR-133a-3p is less than the second threshold value, it can be suggested that the cells induced to differentiate by the second differentiation treatment are not applicable for transplantation. When the measured value of miR-1-3p is equal to or more than the first threshold value or the measured value of miR-133a-3p is equal to or more than the second threshold value, it can be suggested that the cells induced to differentiate by the second differentiation treatment can be applied to transplantation. When the measured value of miR-1-3p is equal to or more than the first threshold value or the measured value of miR-133a-3p is equal to or more than the second threshold value, it can be suggested that at least 70% of the cells induced to differentiate by the second differentiation treatment differentiate into cardiomyocytes.

The first threshold value and the second threshold value are not particularly limited, and can be appropriately set. For example, the threshold values may be set as follows. The first and second differentiation treatments are performed to induce differentiation of PSCs into cardiomyocytes to obtain multiple lots of cells. The supernatant is collected from the liquid medium containing each lot of cells before completion of the operation of inducing differentiation into cardiomyocytes. After completion of the induction of differentiation into cardiomyocytes, the cTnT positive rate is confirmed for each lot of cells. Then, the collected supernatant is classified into a supernatant of a lot having a high cTnT positive rate (for example, 70% or more) and a supernatant of a lot having a low cTnT positive rate (for example, less than 70%). For each supernatant, the measured value of miRNA-3p is acquired. Then, from the obtained measured value, a value capable of distinguishing between the lot having a high cTnT positive rate and the lot having a low cTnT positive rate is obtained, and the value is set as a threshold value. In setting the threshold value, it is preferable to consider sensitivity, specificity, positive predictive value, negative predictive value, and the like.

As described above, the measured value of miRNA-3p in the supernatant collected before completion of the operation of inducing differentiation into cardiomyocytes is an index suggesting the cell differentiation state after completion of the operation. Therefore, the evaluation method of the present embodiment can be rephrased as "a method for obtaining an index for predicting the cell differentiation state".

By the evaluation method of the present embodiment, whether or not the cells induced to differentiate by the second differentiation treatment are well differentiated into cardiomyocytes can be discriminated before completion of the operation of inducing differentiation into cardiomyocytes. When it has been suggested that the cells induced to differentiate by the second differentiation treatment can be applied to transplantation or when it has been suggested that at least 70% of the cells differentiate into cardiomyocytes, a sufficient amount of cardiomyocytes may be obtained by continuing the cell culture. Referring to Fig. 1, culture may be continued, for example, for less than 200 days.

The present invention may include comparing the measured value of miRNA-3p with a threshold value, and determining the cell differentiation state based on the comparison result. In the determining, the cell differentiation state of the cells induced to differentiate by the second differentiation treatment when the operation of inducing differentiation into cardiomyocytes was completed is determined. The cell differentiation state when the operation was completed may be, for example, either a cell differentiation state applicable to transplantation or a cell differentiation state unapplicable for transplantation. Alternatively, the cell differentiation state when the operation was completed may be that at least 70%, at least 75%, at least 80%, at least 85%, at least 90%, or at least 95% of the cells induced to differentiate into cardiomyocytes by the second differentiation treatment.

For example, in a case where the measured value of miR-1-3p is acquired as the measured value of miRNA-3p, when the measured value of miR-1-3p is less than the first threshold value, it can be determined that the cells induced to differentiate by the second differentiation treatment are not applicable for transplantation. When the measured value of miR-1-3p is equal to or more than the first threshold value, it can be determined that the cells induced to differentiate by the second differentiation treatment can be applied to transplantation. When the measured value of miR-1-3p is equal to or more than the first threshold value, it can be determined that at least 70% of the cells induced to differentiate by the second differentiation treatment differentiate into cardiomyocytes. Alternatively, in a case where the measured value of miR-133a-3p is acquired as the measured value of miRNA-3p, when the measured value of miR-133a-3p is less than the second threshold value, it can be determined that the cells induced to differentiate by the second differentiation treatment are not applicable for transplantation. When the measured value of miR-133a-3p is equal to or more than the second threshold value, it can be determined that the cells induced to differentiate by the second differentiation treatment can be applied to transplantation. When the measured value of miR-133a-3p is equal to or more than the second threshold value, it can be determined that at least 70% of the cells induced to differentiate by the second differentiation treatment differentiate into cardiomyocytes. By the above evaluation method using the measured value of miR-1-3p or the measured value of miR-133a-3p, it is possible to early identify cells unapplicable for transplantation or cells applicable to transplantation, and to early determine whether or not culture can be continued. The first threshold value and the second threshold value are as described above.

Alternatively, in a case where the measured value of miR-1-3p and the measured value of miR-133a-3p are acquired as the measured values of miRNA-3p, when the measured value of miR-1-3p is less than the first threshold value and the measured value of miR-133a-3p is less than the second threshold value, it can be determined that the cells induced to differentiate by the second differentiation treatment are not applicable for transplantation. When the measured value of miR-1-3p is equal to or more than the first threshold value and the measured value of miR-133a-3p is equal to or more than the second threshold value, it can be determined that the cells induced to differentiate by the second differentiation treatment can be applied to transplantation. When the measured value of miR-1-3p is equal to or more than the first threshold value and the measured value of miR-133a-3p is equal to or more than the second threshold value, it can be determined that at least 70% of the cells induced to differentiate by the second differentiation treatment differentiate into cardiomyocytes.

Alternatively, in a case where the measured value of miR-1-3p and the measured value of miR-133a-3p are acquired as the measured values of miRNA-3p, when the measured value of miR-1-3p is less than the first threshold value or the measured value of miR-133a-3p is less than the second threshold value, it can be determined that the cells induced to differentiate by the second differentiation treatment are not applicable for transplantation. When the measured value of miR-1-3p is equal to or more than the first threshold value or the measured value of miR-133a-3p is equal to or more than the second threshold value, it can be determined that the cells induced to differentiate by the second differentiation treatment can be applied to transplantation. When the measured value of miR-1-3p is equal to or more than the first threshold value or the measured value of miR-133a-3p is equal to or more than the second threshold value, it can be determined that at least 70% of the cells induced to differentiate by the second differentiation treatment differentiate into cardiomyocytes.

The present invention may further include collecting a supernatant of the liquid medium containing cells induced to differentiate by the first differentiation treatment and before being subjected to the second differentiation treatment, and measuring miR-489-3p in the supernatant. Human miR-489-3p itself is known, and the nucleotide sequence of this miRNA is shown in SEQ ID NO: 3. Gene encoding human miR-489-3p is published in GenBank database under accession number NR_030164. The measured value of miR-489-3p is an index of differentiation into mesodermal cells. The collection timing of the supernatant is not particularly limited as long as a sufficient period of time has elapsed for PSCs to differentiate into mesodermal cells. On the other hand, the first differentiation treatment is completed by medium replacement. When the time has not elapsed so much after medium replacement, it is considered that the miR-489-3p is not sufficiently contained in the supernatant. Therefore, the collection timing of the supernatant may be, for example, a time point at which 1 day or more and 3 days or less have elapsed from the day on which the first differentiation treatment was completed and before the second differentiation treatment is performed.

The timing of collecting the supernatant may be determined, for example, according to the length of the period of the first differentiation treatment. When the period of the first differentiation treatment is 12 hours or more and less than 24 hours, the collection timing of the supernatant may be, for example, a time point at which 2 days or more and 3 days or less have elapsed from the day on which the first differentiation treatment was completed and before the second differentiation treatment is performed. When the period of the first differentiation treatment is 1 day or 2 days, the collection timing of the supernatant may be, for example, a time point at which 1 day or more and 2 days or less have elapsed from the day on which the first differentiation treatment was completed and before the second differentiation treatment is performed. In the example of Fig. 1, the period of the first differentiation treatment is 1 day, and it is preferable to collect the supernatant on day 3 (Day3), that is, 2 days after the completion of the first differentiation treatment.

As a negative control for miRNA measurement, a supernatant before the first differentiation treatment is performed (hereinafter also referred to as "first control sample") may be collected. The first control sample may be a supernatant collected at any time before the first differentiation treatment is performed. The supernatant collected at such a time point contains little miR-489-3p. The first control sample can also be said to be a sample for obtaining a background value for the measurement of miR-489-3p. Preferably, the first control sample is a liquid medium that is removed upon medium replacement to perform the first differentiation treatment. In the example of Fig. 1, the control sample is a liquid medium containing cells before medium replacement on day 0 (Day0). The first control sample can be obtained by recovering a part or all of the liquid medium before removing the liquid medium.

As shown in Example 1 described later, from a liquid medium containing iPS cells subjected to the first differentiation treatment, the supernatants were collected before and after completion of the operation of inducing differentiation into mesodermal cells. In the present specification, the "operation of inducing differentiation into mesodermal cells" includes performing the first differentiation treatment and culturing the cells for a period of time sufficient for the cells in the liquid medium to differentiate into mesodermal cells. After completion of the operation, the cells were subjected to immunofluorescence staining of brachyury T, a mesodermal marker. As a result, the measured value of miR-489-3p was significantly higher in the supernatant collected after completion of differentiation induction than in the supernatant collected before completion of differentiation induction. In addition, in the cells after completion of differentiation induction, the positive rate of brachyury T was high, and most of the iPS cells were differentiated into mesodermal cells. Therefore, based on the measured value of miR-489-3p, information on the cell differentiation state of PSCs into mesodermal cells can be acquired. Examples of such information include information on whether or not PSCs have differentiated into mesodermal cells. For example, when the measured value of miR-489-3p is equal to or more than the third threshold value, it can be suggested that PSCs have differentiated into mesodermal cells. The third threshold value is a threshold value corresponding to the measured value of miR-489-3p.

The present invention may include comparing the measured value of miR-489-3p with a third threshold value, and determining whether or not PSCs have differentiated into mesodermal cells based on the comparison result. For example, when the measured value of miR-489-3p is equal to or more than the third threshold value, PSCs can be determined to differentiate into mesodermal cells. The phrase "differentiate into mesodermal cells" means that a sufficient amount of mesodermal cells was acquired to acquire an amount of cardiomyocytes applicable to transplantation. The phrase "differentiate into mesodermal cells" does not mean that all PSCs have differentiated into mesodermal cells. When the measured value of miR-489-3p is less than the third threshold value, it can be determined that differentiation of PSCs into mesodermal cells is insufficient. Cardiomyocytes differentiate from PSCs through mesodermal cells, thus, when differentiation from PSCs into mesodermal cells is insufficient at this stage, there is a high possibility that cardiomyocytes finally applicable to transplantation cannot be obtained even if differentiation induction is continued. Therefore, when the measured value of miR-489-3p is less than the third threshold value, it can be predicted that cardiomyocytes applicable to transplantation cannot be obtained. When it has been determined that the differentiation from PSCs into mesodermal cells is insufficient or when it has been predicted that cardiomyocytes applicable to transplantation cannot be obtained, the induction of differentiation into cardiomyocytes can be stopped.

The third threshold value is not particularly limited, and can be appropriately set. For example, the threshold value may be set as follows. PSCs are subjected to the first differentiation treatment to obtain differentiation-induced cells. In addition, PSCs cultured without first differentiation treatment are obtained. When the operation of inducing differentiation into mesodermal cells is completed, the supernatant is collected from the liquid medium containing each cell, and the measured value of miR-489-3p is acquired. The brachyury T positive rate is confirmed for each cell. From the obtained measured value, a value capable of distinguishing between the cells subjected to the first differentiation treatment and the cells not subjected to the first differentiation treatment is obtained, and the value is set as a third threshold value. In setting the threshold value, it is preferable to consider sensitivity, specificity, positive predictive value, negative predictive value, and the like.

The present invention also includes a method for producing cardiomyocytes (hereinafter, also referred to as "production method of the present embodiment"). In the production method of the present embodiment, cardiomyocytes are acquired by discriminating that differentiation-induced cells differentiate into cardiomyocytes with high differentiation efficiency based on the measured value of miRNA-3p. In the production method of the present embodiment, first, mesodermal cells are induced to differentiate into cardiomyocytes in a liquid medium by differentiation treatment for inducing mesodermal cells to differentiate into cardiomyocytes. This differentiation treatment is the same as the second differentiation treatment described above. The mesodermal cell is not particularly limited, but is preferably a cell obtained by subjecting PSC to the first differentiation treatment. The liquid medium and culture conditions are as described above.

Next, a supernatant of the liquid medium containing the induced cells is collected, and miRNA-3p in miR-1/133a cluster in the supernatant is measured. The timing and method for collecting the supernatant are the same as those for collecting the supernatant from the liquid medium containing the cells induced to differentiate by the second differentiation treatment. The miRNA measurement method is as described above. Then, the measured value of miRNA-3p in miR-1/133a cluster is compared with the threshold value. When the measured value of miRNA-3p is equal to or more than the threshold value, it is determined that the cells induced to differentiate by the differentiation treatment differentiated into cardiomyocytes at a high ratio as described above. Therefore, cardiomyocytes can be acquired by culturing the cells. Liquid media and culture conditions used for culturing cells induced to differentiate into cardiomyocytes are known. When the measured value of miRNA-3p is less than the threshold value, it is determined that the cells induced to differentiate by the differentiation treatment are not applicable for transplantation as cardiomyocytes as described above. The cell culture can be stopped by the determination.

For example, in a case where the measured value of miR-1-3p is acquired as the measured value of miRNA-3p, when the measured value of miR-1-3p is equal to or more than the first threshold value, cardiomyocytes can be acquired by culturing the cells induced to differentiate by the differentiation treatment. When the measured value of miR-1-3p is less than the first threshold value, the culture can be stopped. Alternatively, in a case where the measured value of miR-133a-3p is acquired as the measured value of miRNA-3p, when the measured value of miR-133a-3p is equal to or more than the second threshold value, cardiomyocytes can be acquired by culturing the cells induced to differentiate by the differentiation treatment. When the measured value of miR-133a-3p is less than the second threshold value, the culture can be stopped. The first threshold value and the second threshold value are as described above.

Alternatively, in a case where the measured value of miR-1-3p and the measured value of miR-133a-3p are acquired as the measured values of miRNA-3p, when the measured value of miR-1-3p is equal to or more than the first threshold value and the measured value of miR-133a-3p is equal to or more than the second threshold value, cardiomyocytes can be acquired by culturing the cells induced to differentiate by the differentiation treatment. When the measured value of miR-1-3p is less than the first threshold value and the measured value of miR-133a-3p is less than the second threshold value, the culture can be stopped.

Alternatively, in a case where the measured value of miR-1-3p and the measured value of miR-133a-3p are acquired as the measured values of miRNA-3p, when the measured value of miR-1-3p is equal to or more than the first threshold value or the measured value of miR-133a-3p is equal to or more than the second threshold value, cardiomyocytes can be acquired by culturing the cells induced to differentiate by the differentiation treatment. When the measured value of miR-1-3p is less than the first threshold value or the measured value of miR-133a-3p is less than the second threshold value, the culture can be stopped.

As the mesodermal cells used in the production method of the present embodiment, cells that are induced to differentiate from PSCs and are discriminated to differentiate into mesodermal cells based on the measured value of miR-489-3p can be used. Specifically, first, PSCs are induced to differentiate into mesodermal cells in a liquid medium by differentiation treatment for inducing PSCs to differentiate into mesodermal cells. This differentiation treatment is the same as the first differentiation treatment described above. The PSC, the liquid medium, and the culture conditions are as described above.

Next, a supernatant of the liquid medium containing the induced cells is collected, and miR-489-3p in the supernatant is measured. The timing and method for collecting the supernatant are the same as those for collecting the supernatant from the liquid medium containing the cells induced to differentiate by the first differentiation treatment. The miRNA measurement method is as described above. Then, the measured value of miR-489-3p is compared with the third threshold value. When the measured value of miR-489-3p is equal to or more than the third threshold value, it is determined that the cells induced to differentiate by the differentiation treatment differentiated into mesodermal cells. Therefore, the cells can be used as mesodermal cells in the production method of the present embodiment. When the measured value of miR-489-3p is less than the third threshold value, it is determined that differentiation of the cells induced to differentiate by the differentiation treatment into mesodermal cells is insufficient, or it is predicted that cardiomyocytes applicable to transplantation cannot be obtained. In this case, the induction of differentiation into cardiomyocytes can be stopped.

The present invention also includes a reagent kit used in a method for evaluating cell differentiation state, a method for determining cell differentiation state, or a method for producing cardiomyocytes (hereinafter, also referred to as "reagent kit"). The reagent kit includes an oligonucleotide primer and/or an oligonucleotide probe for detecting miRNA-3p in miR-1/133a cluster. The reagent kit may further include an oligonucleotide primer and/or an oligonucleotide probe for detecting miR-489-3p. An oligonucleotide primer and/or an oligonucleotide probe for detecting each miRNA may be bound to a solid phase such as a plate or a particle. The reagent kit may include other reagents such as buffers, dNTPs (dATP, dTTP, dGTP and dCTP), reverse transcriptase, and polymerase.

The oligonucleotide primer may be a primer for reverse transcription reaction of each miRNA (hereinafter, also referred to as "RT primer"), or may be a forward primer and/or a reverse primer for amplifying cDNA synthesized from each miRNA (hereinafter, also referred to as "cDNA amplification primer"). The reagent kit may contain both an RT primer and a cDNA amplification primer. When miRNA is measured using an oligonucleotide primer, since miRNA has a short nucleotide length, a stem-loop primer can be used as an RT primer and/or a cDNA amplification primer.

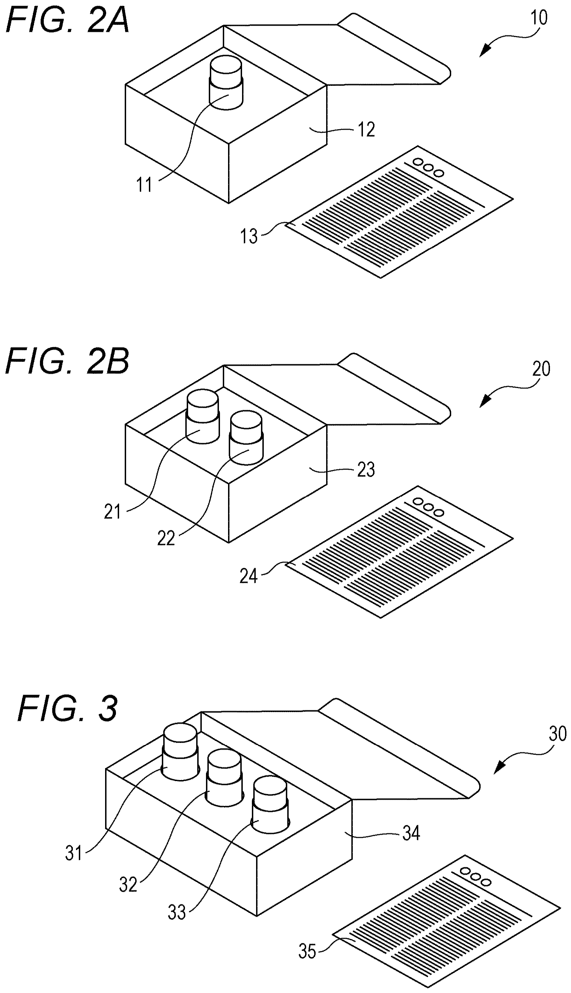

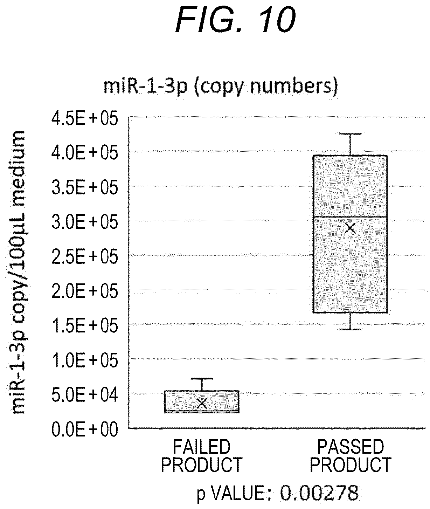

Fig. 2A shows an example of the reagent kit. In Fig. 2A, 10 denotes a reagent kit, 11 denotes a first container containing an oligonucleotide primer and/or an oligonucleotide probe for detecting miR-1-3p, 12 denotes a packing box, and 13 denotes an attached document. In the attached document, nucleotide sequences of primers and/or probes, methods of use such as PCR conditions, storage methods, and the like may be described. The first container may contain an oligonucleotide primer and/or an oligonucleotide probe for detecting miR-133a-3p.

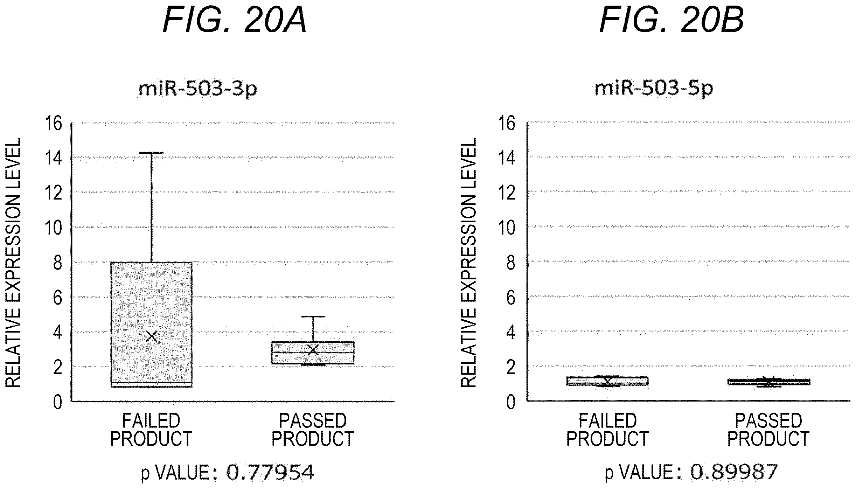

Fig. 2B shows another example of the reagent kit. In Fig. 2B, 20 denotes a reagent kit, 21 denotes a first container containing an oligonucleotide primer and/or an oligonucleotide probe for detecting miR-1-3p, 22 denotes a second container containing an oligonucleotide primer and/or an oligonucleotide probe for detecting miR-133a-3p, 23 denotes a packing box, and 24 denotes an attached document.

Fig. 3 shows another example of the reagent kit. In Fig. 3, 30 denotes a reagent kit, 31 denotes a first container containing an oligonucleotide primer and/or an oligonucleotide probe for detecting miR-1-3p, 32 denotes a second container containing an oligonucleotide primer and/or an oligonucleotide probe for detecting miR-133a-3p, 33 denotes a third container containing an oligonucleotide primer and/or an oligonucleotide probe for detecting miR-489-3p, 34 denotes a packing box, and 35 denotes an attached document.

The present invention includes use of an oligonucleotide primer and/or an oligonucleotide probe for detecting miRNA-3p in miR-1/133a cluster, for producing the reagent kit used in a method for evaluating cell differentiation state, a method for determining cell differentiation state, or a method for producing cardiomyocytes. The present invention includes use of an oligonucleotide primer and/or an oligonucleotide probe for detecting miRNA-3p in miR-1/133a cluster and an oligonucleotide primer and/or an oligonucleotide probe for detecting miR-489-3p, for producing the reagent kit used in a method for evaluating cell differentiation state, a method for determining cell differentiation state, or a method for producing cardiomyocytes.

Hereinbelow, the present invention will be described in detail by examples, but the present invention is not limited to these examples.

### EXAMPLES

### Example 1: Evaluation of mesodermal differentiation by miR489-3p

In the process of inducing differentiation of pluripotent stem cells (PSCs) to acquire cardiomyocytes, it was examined whether miR489-3p can be an index indicating that PSCs differentiated into mesodermal cells by a first differentiation treatment.

### (1) Induction of cell differentiation and collection of supernatant

As PSCs, human iPS cells (253G4 clone and 201B7 clone) were obtained from Center for iPS Cell Research and Application, Kyoto University (CiRA). In Example 1, two groups of iPS cells were prepared, and each group was treated with different efficiency of induction of differentiation into cardiomyocytes. One group included cells subjected to the first and second differentiation treatments, which are generally performed as induction of differentiation into cardiomyocytes. Hereinafter, these cells are also referred to as "Group H" (details of the differentiation treatment will be described later). The other group included cells that were not subjected to the first differentiation treatment but only subjected to the second differentiation treatment. Hereinafter, these cells are also referred to as "Group L". Group L was a group in which the differentiation efficiency was artificially decreased with respect to Group H.

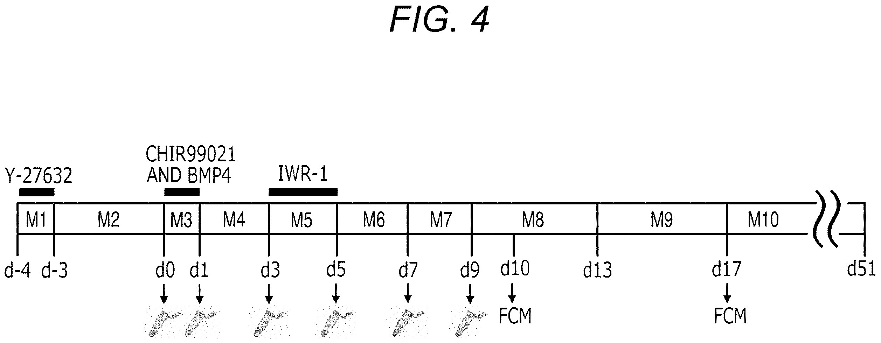

The procedure for inducing differentiation from iPS cells to cardiomyocytes in Example 1 will be described with reference to Fig. 4. Fig. 4 shows a schedule of cell culture and differentiation induction and acquisition timings of supernatant. In Example 1, the day on which the first differentiation treatment was started on iPS cells was defined as "day 0". In Fig. 4, day 0 is denoted as "d0". "M1" to "M10" in the figure indicate various liquid media used in each period. The black bars in the figure indicate the period of time with the liquid medium containing each additive. The arrows in the figure indicate timing when the supernatant was obtained from the liquid medium containing cells. Referring to Fig. 4, from 4 days before day 0 (d-4) to day 0 (d0) was a period for maintenance culture of iPS cells. From day 0 (d0) to day 7 (d7) was a period for inducing the iPS cells to differentiate into cardiomyocytes. From day 7 (d7) onward was a period for maintenance culture of differentiation-induced cardiomyocytes. In order to confirm the proportion of cells differentiated into cardiomyocytes, on day 10 (d10) and day 17 (d17), some of the cells in culture were collected, and cTnT positive cells were detected by the flow cytometry method described later. Details of the operation in each period were as follows.

### (1.1) Maintenance culture

The iPS cells of each clone were maintained and cultured on plates coated with Matrigel (trademark) (Corning). As a liquid medium, StemFit (registered trademark) AS103C (AJINOMOTO CO., INC.) was used. Referring to Fig. 4, the iPS cells (1 × 10⁵ cells each) of each clone were passaged to new plates 4 days before day 0 (d-4). As a liquid medium (M1 in the figure), StemFit AS103C containing 10 µM Y-27632 (FUJIFILM Wako Pure Chemical Corporation) was used. The medium was replaced 3 days before day 0 (d-3), and the cells were cultured until day 0 (d0). The liquid medium after replacement (M2 in the figure) was StemFit AS103C. The iPS cells of each clone were divided into two groups for use as Groups H and L.

### (1.2) First differentiation treatment (induction of differentiation from iPS cells into mesodermal cells)

Referring to Fig. 4, on day 0 (d0), the supernatant (100 µL) was collected from the liquid medium containing cells, and the cells were washed with Dulbecco's PBS (D-PBS) (FUJIFILM Wako Pure Chemical Corporation). To the cells of Group H was added RPMI1640 (FUJIFILM Wako Pure Chemical Corporation) containing 2% B-27 (trademark) supplement (without insulin), 6 µM CHIR99021 (FUJIFILM Wako Pure Chemical Corporation) and 1 ng/mL BMP4 (R&D Systems) as a liquid medium (M3 in the figure), and the cells were cultured until day 1 (d1). To the cells of Group L was added StemFit RPMI1640 containing 2% B-27 (trademark) supplement (without insulin) as a liquid medium (M3 in the figure), and the cells were cultured until day 1 (d1). The subsequent operation was performed on any group. On day 1 (d1), the supernatant (100 µL) was collected from the liquid medium containing cells, and the cells were washed with D-PBS. As a liquid medium (M4 in the figure), RPMI1640 containing 2% B-27 supplement (without insulin) was added, and the cells were cultured until day 3 (d3).

### (1.3) Second differentiation treatment (induction of differentiation from mesodermal cells into cardiomyocytes)

Referring to Fig. 4, on day 3 (d3), the supernatant (100 µL) was collected from the liquid medium containing cells, and the cells were washed with D-PBS. As a liquid medium (MS in the figure), RPMI1640 containing 2% B-27 supplement (without insulin) and 5 µM IWR-1 (Sigma-Aldrich) was added, and the cells were cultured until day 5 (d5). On day 5 (d5), the supernatant (100 µL) was collected from the liquid medium containing cells and the cells were washed with D-PBS. As a liquid medium (M6 in the figure), RPMI1640 containing 2% B-27 supplement (without insulin) was added, and the cells were cultured until day 7 (d7). On day 7 (d7), the supernatant (100 µL) was collected from the liquid medium containing cells, and the medium was replaced. The liquid medium after replacement (M7 in the figure) was MEM α (Thermo Fisher Scientific) containing 5% FBS and 2 mM sodium pyruvate. Then, the cells were cultured until day 9 (d9).

### (1.4) Purification and maintenance of cells after differentiation induction

Referring to Fig. 4, on day 9 (d9), the supernatant (100 µL) was collected from the liquid medium containing cells, and the cells were washed with D-PBS. The cells were dispersed and recovered using a solution containing 0.25% trypsin and 1 mM EDTA. The recovered cells were resuspended in a liquid medium and added to a plate coated with collagen type I (AGC Techno Glass Co., Ltd.). The liquid medium used (M8 in the figure) was MEM α containing 5% FBS. Then, the cells were cultured until day 13 (d13). On day 13 (d13), the medium was replaced. The liquid medium after replacement (M9 in the figure) was StemFit AS501 (AJINOMOTO CO., INC.). The cells were cultured until day 17 (d17). On day 17 (d17), the medium was replaced. The liquid medium after replacement (M10 in the figure) was MEM α containing 5% FBS. Thereafter, the liquid medium was changed every other day, and the cells were maintained and cultured until day 51 (d51).

### (2) Measurement of miR489-3p in supernatant

The miRNA was isolated from the collected supernatant (100 µL) by the Boom method (Boom R. et al., J.Clin.Microbiol.vol. 28, pp. 495-503, 1990) using magnetic beads Sicastar-M (CoreFront). cDNA was synthesized from a part of the obtained miRNA using MultiScribe reverse transcriptase (Applied Biosystems). Reverse transcription reaction was performed by incubating at 16°C for 30 minutes, at 42°C for 30 minutes, and at 85°C for 5 minutes, followed by maintaining at 4°C. Real-time PCR was performed according to the standard TaqMan (trademark) PCR protocol using a hot start ExTaq PCR enzyme (Takara Bio Inc.) and LightCycler (registered trademark) 96 System (Roche). The PCR was performed by heating at 95°C for 10 minutes, followed by 40 cycles of 95°C for 15 seconds and 60°C for 1 minute. The threshold cycle (Ct) value was defined as the number of cycles when the fluorescence intensity exceeded a predetermined threshold value. The Ct values were converted to copy numbers using a calibration curve. The calibration curve was prepared from the measurement results of synthetic miRNAs. Primers and probes for miR489-3p were purchased as TaqMan (trademark) MicroRNA Assay (Thermo Fisher Scientific).

### (3) Immunofluorescence staining of mesodermal markers

Groups Hand L cells on day 1 (d1) were subjected to immunofluorescence staining of brachyury T as a mesodermal marker. Specific operations are as follows. The cells were washed with D-PBS and fixed with 4% paraformaldehyde. The fixed cells were washed with D-PBS and permeabilized with 0.1% Triton (trademark) X. The cells were washed with 0.05% Tween (trademark) 20 in PBS (PBS-T) and blocked with ImmunoBlock (KAC Co., Ltd.) at room temperature for 1 hour. The cells were incubated overnight at 4°C in ImmunoBlock with anti-brachyury-T antibody (Abcam) at 1 : 200. The cells were washed with D-PBS and incubated in ImmunoBlock with a secondary antibody Alexa Fluor (trademark) 488 labeled anti-rabbit IgG antibody (Thermo Fisher Scientific) at room temperature for 2 hours. The cells were washed twice with D-PBS, and nuclei were stained with 5 µg/mL Hoechst 33342 (Thermo Fisher Scientific). The cells were washed with D-PBS and observed with a fluorescence microscope BZ-X710 (Keyence Corporation). Based on the observation results, the ratio of brachyury T-positive cells was calculated.

### (4) Detection of cTnT positive cells

PE-Labeled anti-cardiac troponin T (cTnT) antibody was bound to the cells of Groups H and L on day 10, and flow cytometry (FCM) analysis was performed to detect cTnT positive cells. Specific operations are as follows. After washing the cells with D-PBS, the cells were dispersed and recovered using a solution containing 0.25% trypsin and 1 mM EDTA. The cells were fixed with 4% paraformaldehyde. The fixed cells were washed with D-PBS and permeabilized with 0.1% Triton (trademark) X. The cells were washed with PBS-T and incubated in ImmunoBlock with PE-labeled anti-cTnT antibody (Miltenyi Biotec) or PE-labeled anti-REA control antibody (Miltenyi Biotec) for 15 to 20 min in the dark. The cells were washed with D-PBS and analyzed on a Gallios flow cytometer (Beckman Coulter, Inc.). The cTnT positive rate was calculated based on the number of detected cTnT positive cells.

### (5) Results

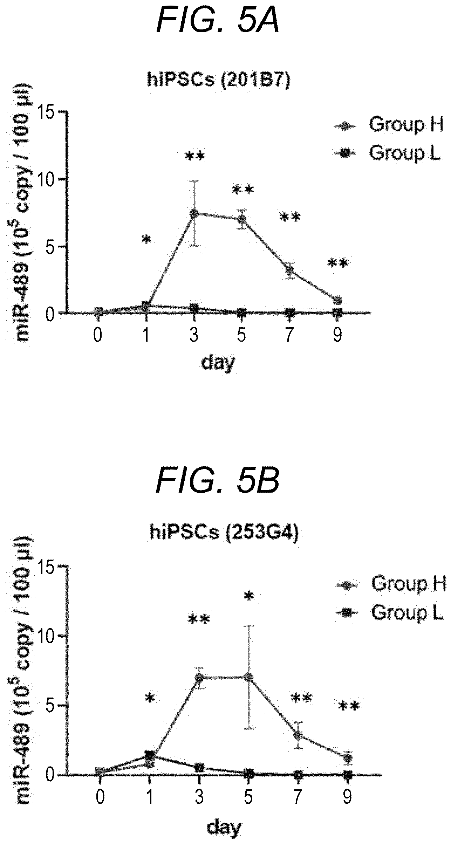

Figs. 5A and 5B show the copy number of miR-489-3p in 100 µL of the supernatant obtained in the process of inducing differentiation of iPS cells of each clone (n=3, experiments were performed independently for each clone). In the figure, "*" indicates p < 0.05, and "**" indicates p < 0.01. Error bars show standard deviation (SD). As can be seen from Fig. 5A, in Group H of clone 201B7, the expression level of miR-489-3p hardly changed on day 1, but the expression level of miR-489-3p sharply increased on day 3. Then, the expression level of miR-489-3p decreased after day 5, and expression of miR-489-3p almost disappeared on day 9. On the other hand, in Group L, no significant increase in the expression level of miR-489-3p was observed between day 0 and day 9. Referring to Fig. 5B, similar results were obtained in Groups H and L of clone 253G4.

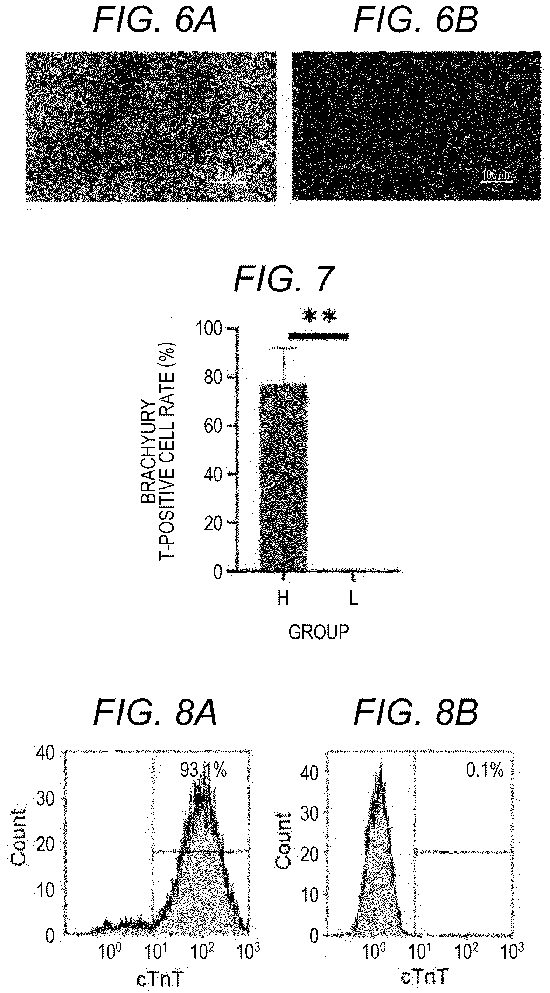

For Groups H and L, an example of an image of immunofluorescently-stained cells is shown in Fig. 6. In the figure, the scale bar indicates 100 µm. Fig. 7 shows the ratio of brachyury T-positive cells in Groups H and L (experiments were performed independently for n=3, 253G4 clones). In Fig. 7, "**" indicates p < 0.01. Error bars show SD. As can be seen from these figures, the cells of Group H were mostly brachyury T-positive cells. On the other hand, in Group L, no brachyury T-positive cells were detected. Since brachyury T is a mesodermal marker, these results indicated that the cells of Group H differentiated into mesodermal cells on day 1. On the other hand, it was shown that the cells of Group L did not differentiate into mesodermal cells.

Fig. 8 shows a graph with fluorescence signal intensity (in the figure, denoted as "cTnT") and count (number of cells) as biaxes for Groups Hand L. The cells with high fluorescence signal intensity were cTnT positive cells. The cTnT positive rate of the cells of Group H was 93.1%, and the cTnT positive rate of the cells of Group L was 0.1%. Since cTnT is a cardiomyocyte marker, these results showed that the cells of Group H differentiated into cardiomyocytes on day 10. On the other hand, it was shown that the cells of Group L did not differentiate into cardiomyocytes.

As described above, in Group H, most of the iPS cells were induced to differentiate into mesodermal cells in one day from the first differentiation treatment start, and the expression level of miR-489-3p increased on day 3 from the first differentiation treatment start. On the other hand, in Group L not subjected to the first differentiation treatment, differentiation into mesodermal cells was not observed, and the expression level of miR-489-3p was not increased. In Group H, the expression level of miR-489-3p decreased after starting the second differentiation treatment on day 5. On day 10, most of the mesodermal cells in Group H differentiated into cardiomyocytes. This suggested that the expression of miR-489-3p decreased as mesodermal cells were induced to differentiate into cardiomyocytes. These results suggested that miR-489-3p is an index indicating that PSCs differentiated into mesodermal cells. Immunofluorescence staining with brachyury T consumes valuable PSCs, but it was found that differentiation of PSCs into mesodermal could be evaluated non-destructively by miR-489-3p measurement in the supernatant.

### Example 2: Evaluation of myocardial differentiation by miR-1-3p and miR-133a-3p

In the process of inducing differentiation of PSCs to acquire cardiomyocytes, it was examined whether miR-1-3p and miR-133a-3p can be indices of differentiation into cardiomyocytes. For comparison, various miRNAs known to be associated with cardiomyocyte differentiation were also measured.

### (1) Induction of cell differentiation, collection of supernatant, and classification of acquired cells

Human iPS cells were subjected to the first and second differentiation treatments under clinical-grade mass culture conditions to obtain differentiation-induced cells (11 lots). The procedure of differentiation induction in Example 2 followed the method of Group H in Example 1 (see Fig. 4). On days 3 and 7, the supernatants (100 µL) were collected from the liquid media containing each lot of cells. On days 10 and 17, a part of each lot of cells was collected, and the cTnT positive rate was calculated by FCM analysis. On day 10, cells of lots having a cTnT positive rate of 70% or more were classified as "passed product", and cells of lots having a cTnT positive rate of less than 70% were classified as "failed product". Table 2 shows the cTnT positive rate of each lot of cells on day 10 (d10) and day 17 (d17).

**[Table 2]**

| Passed product Lot number | cTnT Positive rate (%) | | | Failed product Lot number | cTnT Positive rate (%) | |
|---|---|---|---|---|---|---|
| | d10 | d17 | | | d10 | d17 |
| HS129-2 | 92.14 | 98.05 | | HS022 | 28.53 | 91.27 |
| HS-N | 92.35 | 99.46 | | HS023 | 47.29 | 98.93 |
| HS119 | 93.39 | 99.47 | | HS052 | 49.21 | 37.3 |
| HS129-1 | 93.57 | 98.05 | | HS043 | 55.81 | 46.09 |
| HS148 | 97.75 | 98.93 | | HS084#2 | 64.42 | 35.72 |
| HS149 | 98.18 | 99.2 | | | | |

### (2) Measurement of miRNA in supernatant

In the same manner as in Example 1, miRNAs in the supernatants on day 3 and day 7 were measured. The miRNAs measured were miR-1-3p, miR-1-5p, miR-133a-3p, miR-133a-5p, miR-23b-3p, miR-23b-5p, miR-26b-3p, miR-26b-5p, miR-125b-3p, miR-125b-5p, miR-145-3p, miR-145-5p, miR-499a-3p, miR-499a-5p, miR-503-3p, and miR-503-5p. miRNAs other than miR-1 and miR-133a were known to be associated with cardiomyocyte differentiation. miR-23b was known to be involved in myocardial differentiation. miR-26b was known to be involved in myocardial differentiation and Wnt signaling system. miR-125b was known to be involved in early differentiation of myocardium together with LIN28. miR-145 was known to be involved in myocardial differentiation and myocardial related genes. miR-499a was known to be involved in myocardial differentiation and form a cluster with miR-208. miR-503 was known to be involved in myocardial differentiation and myocardial related genes and form a cluster with miR-322. Measurement of the miRNA was performed in the same manner as in Example 1. Primers and probes for each miRNA were purchased as TaqMan (trademark) MicroRNA Assay (Thermo Fisher Scientific).

### (3) Results

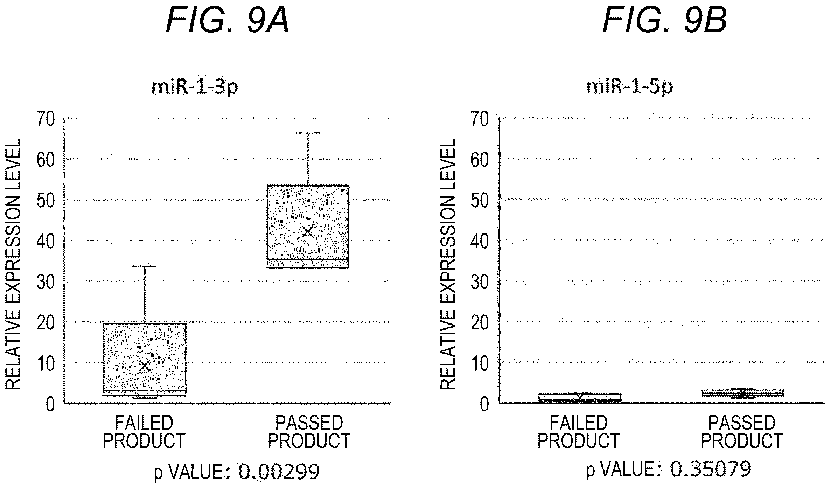

In Figs. 9 to 20, the relative expression levels of each miRNA in 100 µL of the supernatant were shown (passed product n=6, failed product n=5, experiment was performed independently for each lot). The relative expression level was calculated by the following formula. (Relative expression level) = 2^{(Ct value when miRNA in supernatant on day 3 was measured) - (Ct value when miRNA in supernatant on day 7 was measured)}

As described above, in the lots classified as passed products, the proportion of cells differentiated into cardiomyocytes was as high as 90% or more at the time point of day 10. On the other hand, in the lots classified as failed products, the proportion of cells differentiated into cardiomyocytes was low. When the relative expression level of miRNA shows a significant difference between the passed product and the failed product, it can be said that the miRNA is an index that enables discrimination between the lots in which the proportion of cells differentiated into cardiomyocytes is high and the lots in which the proportion is low. Hereinafter, the measurement results of each miRNA will be described.

Referring to Fig. 9A, the relative expression level of miR-1-3p was high in the passed product and low in the failed product. The difference in the relative expression levels between the passed product and the failed product was significant (p=0.00299). On the other hand, referring to Fig. 9B, the relative expression level of miR-1-5p was low in both the passed product and the failed product, and showed no significant difference (p=0.35079). From these results, it was shown that it was possible to discriminate between the passed product and the failed product by the relative expression level of miR-1-3p, but such discrimination was not possible by the relative expression level of miR-1-5p. Fig. 10 shows a box plot obtained by converting the relative expression level in Fig. 9A into copy number. As can be seen from Fig. 10, displaying the measured value of miR-1-3p by copy number more prominently indicated the difference between the passed product and the failed product (p=0.00278). Thus, the measurement of miR-1-3p in the supernatant up to day 7 gave the same results as the FCM analysis performed on day 10. Therefore, it was suggested that miR-1-3p is an index of differentiation into cardiomyocytes.

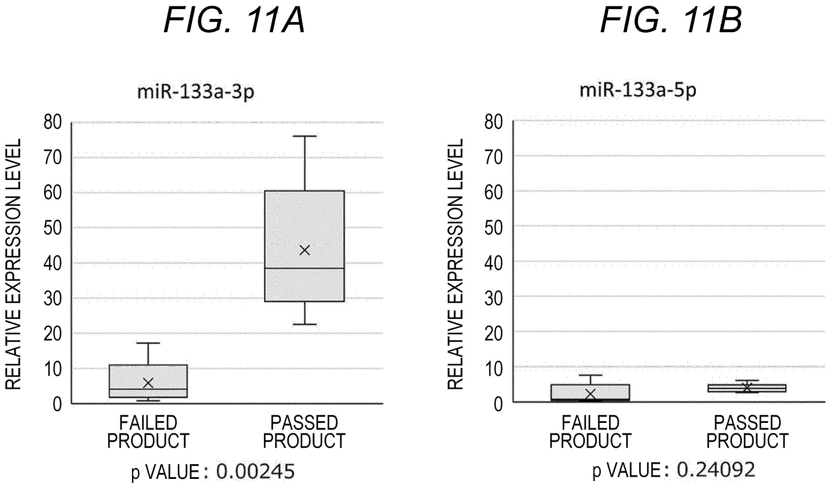

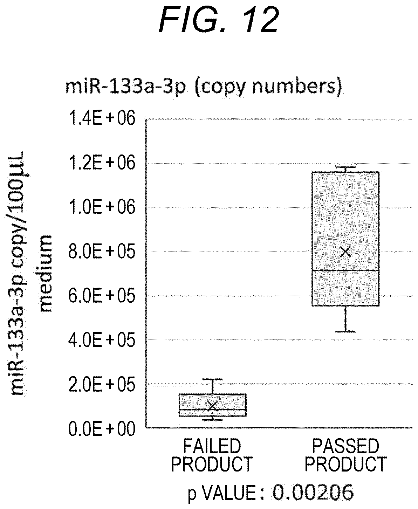

The measurement result of miR-133a-3p was similar to that of miR-1-3p. Referring to Fig. 11A, the relative expression level of miR-133a-3p was high in the passed product and low in the failed product. The difference in the relative expression levels between the passed product and the failed product was significant (p=0.00245). On the other hand, referring to Fig. 11B, the relative expression level of miR-133a-5p was low in both the passed product and the failed product, and showed no significant difference (p=0.24092). Fig. 12 shows a box plot obtained by converting the relative expression level in Fig. 11A into copy number. As can be seen from Fig. 12, displaying the measured value of miR-133a-3p by copy number more prominently indicated the difference between the passed product and the failed product (p=0.00206). Therefore, it was possible to discriminate between the passed product and the failed product by the relative expression level of miR-133a-3p. It was suggested that miR-133a-3p is an index of differentiation into cardiomyocytes.

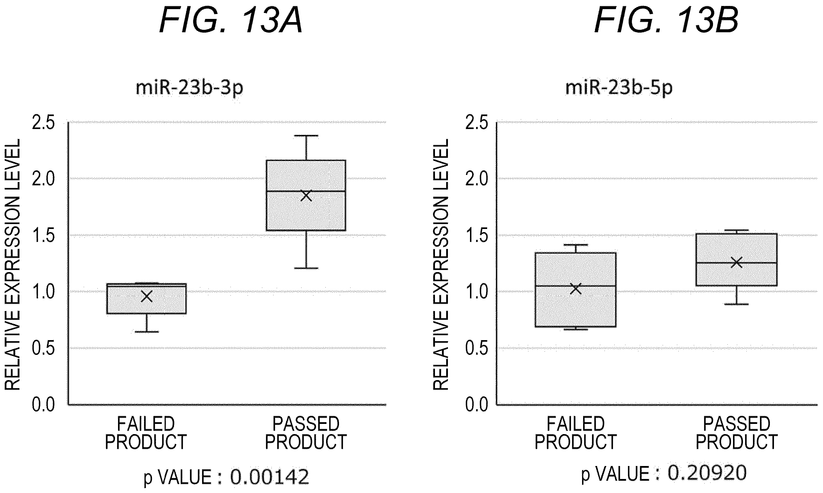

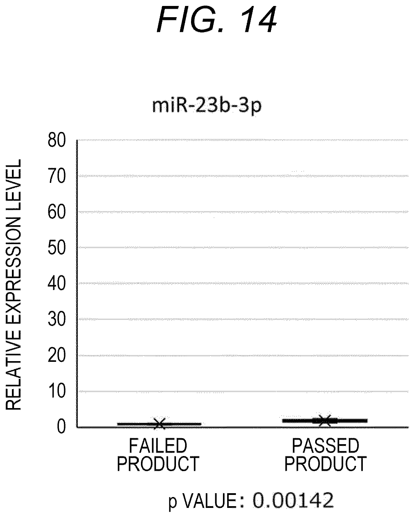

Referring to Fig. 13A, the relative expression level of miR-23b-3p was high in the passed product and low in the failed product. The difference in the relative expression levels between the passed product and the failed product was significant (p=0.00142). On the other hand, referring to Fig. 13B, the relative expression level of miR-23b-5p showed no significant difference between the passed product and the failed product (p=0.20920). From these results, it was shown that it was possible to discriminate between the passed product and the failed product by the relative expression level of miR-23b-3p, but such discrimination was not possible by the relative expression level of miR-23b-5p. However, as compared with miR-1-3p and miR-133a-3p, the relative expression level of miR-23b-3p in the passed product was small. In this regard, for example, the box plot of Fig. 13A was re-created by changing the maximum value on the vertical axis to 80 so that Fig. 13A can be compared with Fig. 11A. The re-created box plot is shown in Fig. 14. As can be seen from Fig. 14, the relative expression level of miR-23b-3p was displayed low in both the passed product and the failed product. This was due to the fact that the expression level of miR-23b-3p in the passed product was not significantly different between day 3 and day 7. Therefore, it was suggested that miR-1-3p and miR-133a-3p can more clearly discriminate between the passed product and the failed product than miR-23b-3p.

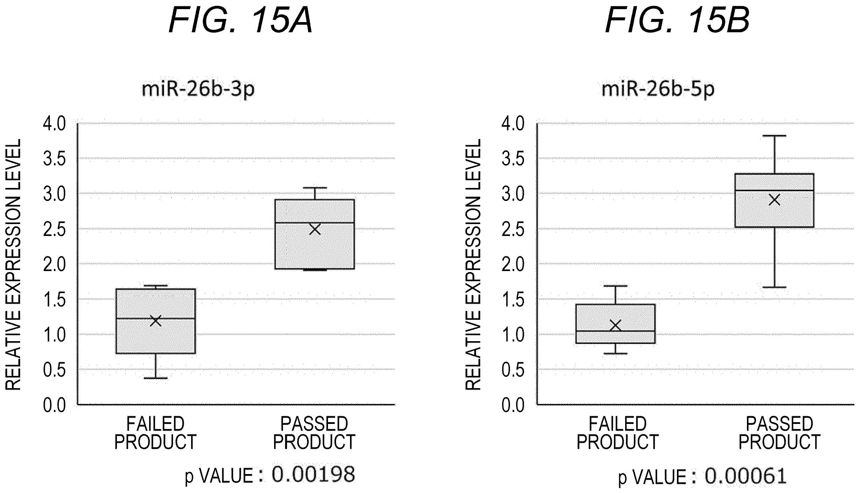

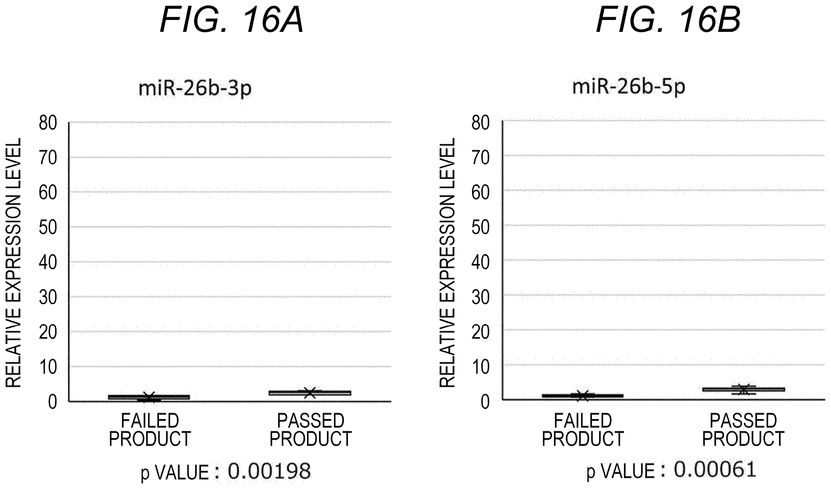

Referring to Fig. 15A, the relative expression level of miR-26b-3p was high in the passed product and low in the failed product. The difference in the relative expression levels between the passed product and the failed product was significant (p=0.00198). The measurement result of miR-26b-5p was similar to that of miR-26b-3p. Referring to Fig. 15B, the relative expression level of miR-26b-5p showed a significant difference between the passed product and the failed product (p=0.00061). From these results, it was shown that it is possible to discriminate between the passed product and the failed product by the relative expression levels of miR-26b-3p and miR-26b-5p. However, as compared with miR-1-3p and miR-133a-3p, the relative expression levels of miR-26b-3p and miR-26b-5p in the passed product were small. The box plots of Figs. 15A and 15B were re-created by changing the maximum value on the vertical axis to 80. These are shown in Figs. 16A and 16B. As can be seen from these figures, the relative expression levels of miR-26b-3p and miR-26b-5p were displayed low in both the passed product and the failed product. Therefore, it was suggested that miR-1-3p and miR-133a-3p can more clearly discriminate between the passed product and the failed product than miR-26b-3p and miR-26b-5p.

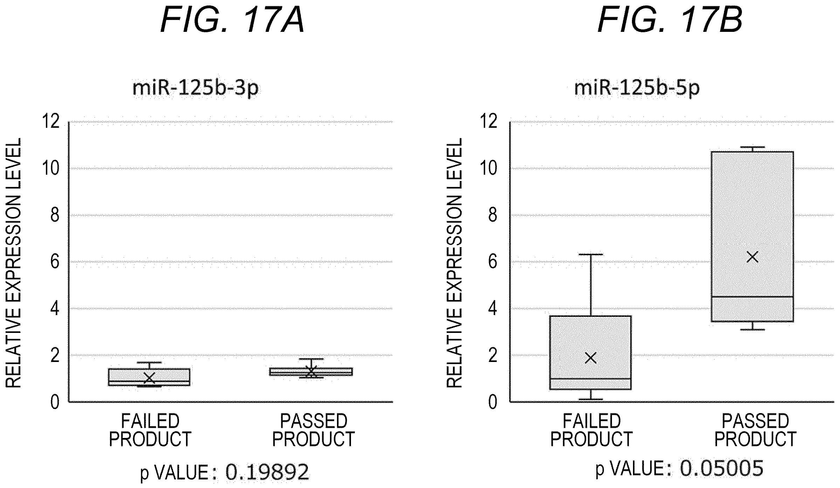

Referring to Fig. 17A, the relative expression level of miR-125b-3p was low in both the passed product and the failed product, and showed no significant difference (p=0.19892). On the other hand, referring to Fig. 17B, the relative expression level of miR-125b-5p was high in the passed product and low in the failed product. However, miR-125b-5p did not show significant differences in relative expression levels (p=0.05005) to the extent that the passed product and the failed product could be clearly distinguished. From these results, it was shown that the passed product and the failed product cannot be discriminated from each other by the relative expression levels of miR-125b-3p and miR-125b-5p.

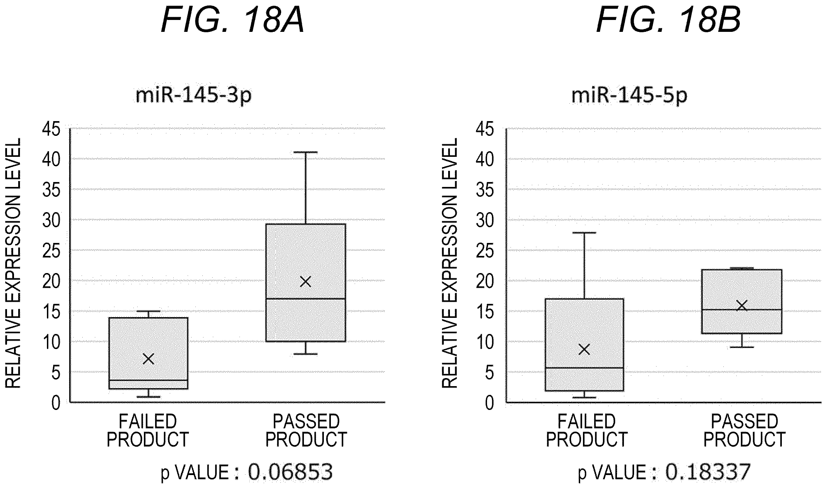

Referring to Fig. 18A, the relative expression level of miR-145-3p was high in the passed product and low in the failed product. However, miR-145-3p did not show significant differences in relative expression levels (p=0.06853) to the extent that the passed product and the failed product could be clearly distinguished. Referring to Fig. 18B, the relative expression level of miR-145-5p showed no significant difference between the passed product and the failed product (p=0.18337). From these results, it was shown that the passed product and the failed product cannot be discriminated from each other by the relative expression levels of miR-145-3p and miR-145-5p.

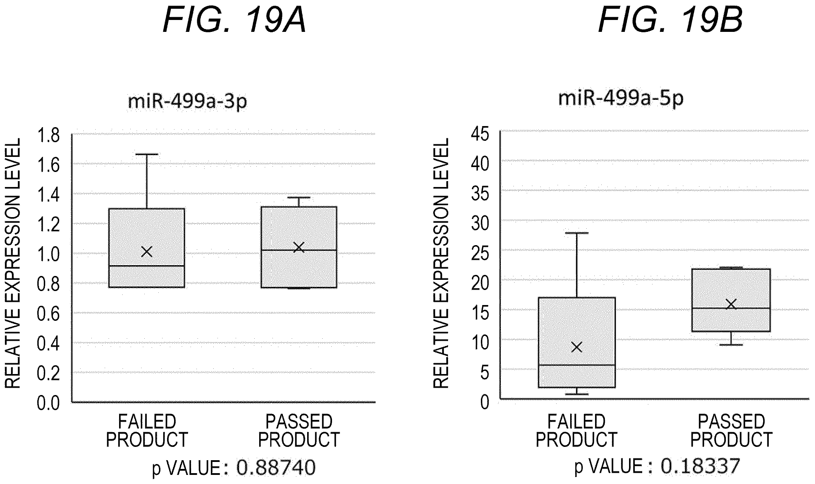

Referring to Figs. 19A and 19B, none of the relative expression levels of miR-499a-3p and miR-499a-5p showed a significant difference between the passed product and the failed product (p=0.88740 and 0.18337). From these results, it was shown that the passed product and the failed product cannot be discriminated from each other by the relative expression levels of miR-499a-3p and miR-499a-5p.

Referring to Figs. 20A and 20B, none of the relative expression levels of miR-503-3p and miR-503-5p showed a significant difference between the passed product and the failed product (p=0.77954 and 0.89987). From these results, it was shown that the passed product and the failed product cannot be discriminated from each other by the relative expression levels of miR-503-3p and miR-503-5p.

## Claims

1. A method for evaluating cell differentiation state, comprising:
inducing differentiation of pluripotent stem cells into cardiomyocytes in a liquid medium by inducing differentiation of pluripotent stem cells into mesodermal cells as a first differentiation treatment and by inducing differentiation of the mesodermal cells into cardiomyocytes as a second differentiation treatment;
collecting a supernatant of the liquid medium comprising differentiation-induced cells of the second differentiation treatment; and
obtaining measurement value of miRNA-3p from miR-1/133a cluster in the supernatant,
wherein the miRNA-3p is at least one selected from a group consisting of miR-1-3p and miR-133a-3p, and
wherein the obtained measurement value of miRNA-3p serves as an index of cell differentiation state of the differentiation-induced cells.

2. The method according to claim 1, wherein the miRNA-3p is the miR-1-3p, and wherein when the obtained measurement value of miR-1-3p is less than a first threshold value, it is suggested that the differentiation-induced cells are not applicable for transplantation; or
wherein the miRNA-3p is the miR-133a-3p, and wherein when the obtained measurement value of miR-133a-3p is less than a second threshold value, it is suggested that the differentiation-induced cells are not applicable for transplantation.

3. The method according to claim 1, wherein the miRNA-3p is the miR-1-3p and the miR-133a-3p, and
wherein when the obtained measurement value of miR-1-3p is less than a first threshold value and the obtained measurement value of miR-133a-3p is less than a second threshold value, it is suggested that the differentiation-induced cells are not applicable for transplantation.

4. The method according to claim 1, wherein
the miRNA-3p is the miR-1-3p and the miR-133a-3p, and
wherein when the obtained measurement value of miR-1-3p is less than a first threshold value or the obtained measurement value of miR-133a-3p is less than a second threshold value, it is suggested that the differentiation-induced cells are not applicable for transplantation.

5. The method according to claim 1, further comprising collecting a supernatant of the liquid medium comprising cells induced differentiation by the first differentiation treatment and before being subjected to the second differentiation treatment, and obtaining measurement value of miR-489-3p in the supernatant, wherein the obtained measurement value of miR-489-3p serves as an index of cell differentiation state of pluripotent stem cells into mesodermal cells.

6. The method according to claim 5, wherein when the obtained measurement value of miR-489-3p is equal to or more than a third threshold value, it is suggested that the cells induced differentiation by the first differentiation treatment have differentiated into mesodermal cells.

7. A method for determining cell differentiation state comprising:
inducing differentiation of pluripotent stem cells into mesodermal cells in a liquid medium as a first differentiation treatment and inducing differentiation of the mesodermal cells into cardiomyocytes as a second differentiation treatment;
collecting a supernatant of the liquid medium comprising differentiation-induced cells of the second differentiation treatment;
obtaining measurement value of miRNA-3p from miR-1/133a cluster in the supernatant; and
determining that the differentiation-induced cells are not applicable for transplantation when the obtained measurement value of miRNA-3p is less than a threshold value, wherein
the miRNA-3p is at least one selected from miR-1-3p and miR-133a-3p.

8. The method according to claim 7, wherein
the miRNA-3p is the miR-1-3p, and the threshold value is a first threshold value, and
in the determining, when the obtained measurement value of miR-1-3p is less than the first threshold value, it is determined that the differentiation-induced cells are not applicable for transplantation.

9. The method according to claim 7, wherein
the miRNA-3p is the miR-133a-3p, and the threshold value is a second threshold value, and
in the determining, when the obtained measurement value of miR-133a-3p is less than the second threshold value, it is determined that the differentiation-induced cells are not applicable for transplantation.

10. The method according to claim 7, wherein
the miRNA-3p is the miR-1-3p and the miR-133a-3p, and the threshold values are a first threshold value and a second threshold value, and
in the determining, when the obtained measurement value of miR-1-3p is less than the first threshold value and the obtained measurement value of miR-133a-3p is less than the second threshold value, it is determined that the differentiation-induced cells are not applicable for transplantation.

11. The method according to claim 7, wherein
the miRNA-3p is the miR-1-3p and the miR-133a-3p, and the threshold values are a first threshold value and a second threshold value, and
in the determining, when the obtained measurement value of miR-1-3p is less than the first threshold value or the obtained measurement value of miR-133a-3p is less than the second threshold value, it is determined that the differentiation-induced cells are not applicable for transplantation.

12. The method according to any of claims 1 to 11, wherein the first differentiation treatment comprises using a liquid medium comprising at least one of a substance that activates a BMP signaling or a substance that activates a Wnt signaling.

13. The method according to any of claims 1 to 11, wherein the second differentiation treatment comprises using a liquid medium comprising a substance that inhibits a Wnt signaling.

14. The method according to claim 7, further comprising:
collecting a supernatant of the liquid medium comprising cells induced differentiation by the first differentiation treatment and before being subjected to the second differentiation treatment, and obtaining measurement value of miR-489-3p in the supernatant; and
determining that the pluripotent stem cells have differentiated into mesodermal cells when the obtained measurement value of miR-489-3p is equal to or more than a third threshold value, and/or
determining that differentiation of the pluripotent stem cells into mesodermal cells is insufficient when the obtained measurement value of miR-489-3p is less than a third threshold value.

15. A method for producing cardiomyocytes comprising:
(1) inducing differentiation of mesodermal cells into cardiomyocytes in a liquid medium by differentiation treatment for inducing differentiation of mesodermal cells into cardiomyocytes;
(2) collecting a supernatant of the liquid medium comprising cells induced differentiation in the step (1);
(3) obtaining measurement value of miRNA-3p from miR-1/133a cluster in the supernatant collected in the step (2); and
(4) culturing the cells induced differentiation in the step (1) to acquire cardiomyocytes when the obtained measurement value of miRNA-3p is equal to or more than a threshold value, wherein
the miRNA-3p is at least one selected from miR-1-3p and miR-133a-3p.

16. The method according to claim 15, wherein in the step (4), when the obtained measurement value of miRNA-3p is less than a threshold value, culturing of the cells induced differentiation in the step (1) is stopped.

17. The production method according to claim 15, comprising:
(i) inducing differentiation of pluripotent stem cells into mesodermal cells in a liquid medium by differentiation treatment for inducing differentiation of pluripotent stem cells into mesodermal cells;
(ii) collecting a supernatant of the liquid medium comprising cells induced differentiation in the step (i); and
(iii) obtaining measurement value of miR-489-3p in the supernatant collected in the step (ii), wherein
when the obtained measurement value of miR-489-3p is equal to or more than a third threshold value, the cells of the step (ii) are used as the mesodermal cells of the step (1).

18. The method according to claim 17, wherein when the obtained measurement value of miR-489-3p is less than a threshold value, culturing of the cells induced differentiation in the step (ii) is stopped.
